# FASCICULE DE BREVET EUROPEEN

(11) **EP 1 224 310 B1**
(45) Date de publication et mention de la délivrance du brevet: **28.02.2007**
(21) Numéro de dépôt: 00967960.6
(22) Date de dépôt: 05.10.2000
(51) Int. Cl.: C12N 15/861, C12N 15/10, C12N 5/10, A61K 48/00

(54) **PREPARATION D'ADENOVIRUS RECOMBINANTS ET DE BANQUES ADENOVIRALES**
HERSTELLUNG VON REKOMBINANTEN ADENOVIREN UND VON ADENOVIRUS-GENBANKEN
RECOMBINANT ADENOVIRUSES PREPARATION AND ADENOVIRUS BANKS

(30) Priorité: 07.10.1999 FR 9912521; 01.12.1999 US 168356 P
(43) Date de publication de la demande: 24.07.2002
(73) Titulaire: Aventis Pharma S.A., 92160 Antony (FR)
(72) Inventeur: Robert, Jean-Jacques, 92330 Sceaux (FR)
(74) Mandataire: Lecca, Patricia S.
(86) Numéro de dépôt international: PCT/FR2000/002774
(87) Numéro de publication internationale: WO 2001/025463

(56) Documents cités:
- FR-A- 2 718 150
- FR-A- 2 730 411
- FR-A- 2 730 504
- FR-A- 2 755 975
- US-A- 5 922 576
- BETT A. J. ET AL.: "AN EFFICIENT AND FLEXIBLE SYSTEM FOR CONSTRUCTION OF ADENOVIRUS VECTORS WITH INSERTIONS OR DELETIONS IN EARLY REGIONS 1 AND 3" PROCEEDINGS OF THE NATIONAL ACADEMY OF SCIENCES OF USA, vol. 91, 1 septembre 1994 (1994-09-01), pages 8802-8806, XP002004337 ISSN: 0027-8424
- HE T.-C. ET AL.: "A simplified system for generating recombinant adenoviruses." PROC. NATL. ACAD. SCI. USA, vol. 95, mars 1998 (1998-03), pages 2509-2514, XP002144832
- CHARTIER C. ET AL.: "Efficient generation of recombinant adenovirus vectors by homologous recombination in E. coli." JOURNAL OF VIROLOGY, vol. 70, no. 7, 1996, pages 4805-4810, XP002144833
- CROUZET J. ET AL.: "RECOMBINATIONAL CONSTRUCTION IN ESCHERICHIA COLI OF INFECTIOUS ADENOVIRAL GENOMES" PROCEEDINGS OF THE NATIONAL ACADEMY OF SCIENCES OF USA, vol. 94, 1 février 1997 (1997-02-01), pages 1414-1419, XP000619777 ISSN: 0027-8424 cité dans la demande

## Description

La présente invention concerne des compositions et méthodes pour la préparation d'adénovirus recombinants. Les adénovirus produits peuvent être utilisés pour le transfert et/ou l'expression de gènes dans des cellules, in vitro, ex vivo ou in vivo, ou également en génomique fonctionnelle. En particulier, la présente invention concerne des méthodes particulièrement efficaces pour la production de banques adénovirales et l'utilisation de telles banques en génomique fonctionnelle. Elle concerne également des plasmides utilisés pour la construction de ces adénovirus, les cellules contenant ces plasmides, et des kits comportant ces plasmides, cellules et ou banques adénovirales.

Les adénovirus présentent certaines propriétés intéressantes pour le transfert et/ou l'expression de gènes dans des cellules. Notamment, ils ont un spectre d'hôte assez large, sont capables d'infecter des cellules quiescentes, ont une capacité de clonage importante et ne s'intègrent pas au génome de la cellule infectée. En outre, ils n'ont pas été associés à ce jour à des pathologies importantes chez l'homme et ont donc été utilisés pour transférer des gènes d'intérêt dans différents tissus humains, in vitro ou in vivo, comme le muscle (Ragot et al., Nature 361 (1993) 647), le foie (Jaffe et al., Nature genetics 1 (1992) 372), le système nerveux (Akli et al., Nature genetics 3 (1993) 224), les tumeurs, le muscle lisse, etc. Ces mêmes propriétés font du vecteur adénoviral un outil de choix pour exploiter les données issues de la génomique.

La génomique fonctionnelle s'entend comme le champs de sciences s'attachant à exploiter les génomes ou les données du génome d'organismes divers pour les traduire en terme de fonction. Compte tenu de leurs propriétés, les vecteurs adénoviraux portant une séquence exogène peuvent être utilisés pour déterminer la fonction de cette séquence dans divers modèles expérimentaux. En particulier, les vecteurs adénoviraux pourraient être utilisés pour étudier une cible thérapeutique (au sens ou l'entend l'industrie pharmaceutique) dans un modèle cellulaire in vitro ou in vivo chez l'animal. Lorsque cette séquence est connue et caractérisée, les vecteurs adénoviraux peuvent servir à la validation fonctionnelle de cette cible. Plus largement, dans le contexte appelé génomique fonctionnelle, le vecteur adénoviral de transfert de gène pourrait constituer un outil performant pour identifier la fonction d'une séquence nucléique dans un modèle expérimental eucaryote, sans avoir au préalable d'information particulière sur la nature ou la fonction de cette séquence, y compris dans des situations où de nombreuses séquences doivent être étudiées en masse.

Cependant, l'exploitation industrielle, thérapeutique ou en génomique fonctionnelle des adénovirus est encore limitée, notamment par les méthodes actuelles de préparation de ces virus recombinants. En particulier, les méthodes disponibles actuellement ne permettent de produire, de manière simple, rapide et clonale, des populations d'adénovirus incorporant des acides nucléiques hétérologues, surtout lorsque de nombreux acides nucléiques hétérologues doivent être étudiés, comme c'est le cas en génomique fonctionnelle. La présente invention apporte précisément une solution à ces problèmes. La présente invention décrit en effet de nouveaux outils et de nouvelles méthodes pour la construction d'adénovirus recombinants. L'invention décrit notamment des constructions génétiques (plasmides), des cellules, et des protocoles permettant de produire des adénovirus avec une rapidité et une qualité importantes. L'invention permet plus particulièrement de réaliser des banques adénovirales comprenant un nombre élevé d'acides nucléiques hétérologues.

L'invention est également relative à des méthodes et outils permettant la production de tels adénovirus, en particulier de telles banques, notamment avantageusement la construction simultanée d'adénovirus recombinants à partir de banques d'acides nucléiques.

Les adénovirus sont des virus à ADN double brin linéaire d'une taille de 36 kb environ. Leur génome comprend notamment une séquence inversée répétée (ITR) à chaque extrémité, une séquence d'encapsidation (Psi), des gènes précoces et des gènes tardifs. Les principaux gènes précoces sont contenus dans les régions E1, E2, E3 et E4. Parmi ceux-ci, les gènes contenus dans la région E1 sont nécessaires à la propagation virale. La région E4 est également importante dans la régulation de la réplication du génome adénoviral. Les principaux gènes tardifs sont contenus dans les régions L1 à L5. Le génome de l'adénovirus Ad5 a été entièrement séquencé et est accessible sur base de données (voir notamment Genbank M73260). De même des parties, voire la totalité d'autres génomes adénoviraux, humains ou animaux (Ad2, Ad7, Ad12, CAV-2, etc.) ont également été séquencées.

Par ailleurs, comme indiqué ci-avant, les adénovirus ont déjà été utilisés pour le transfert de gènes in vivo. A cet effet, différents vecteurs dérivés des adénovirus ont été préparés, incorporant différents gènes (β-gal, OTC, α-1AT, cytokines, enzymes, facteurs de croissance, etc.). Dans chacune de ces constructions, le génome de l'adénovirus a été modifié de manière à le rendre incapable de réplication et/ou propagation autonome après transfert génique. Ainsi, les constructions décrites dans l'art antérieur sont des adénovirus délétés d'une ou plusieurs régions choisies parmi E1 , E2, E3, E4, pIX, Iva2, etc. Des constructions particulières sont dépourvues de la région E1, des régions E1 et E3, des régions E1 et E4, des régions E1, E4 et E3, ou encore de la totalité des régions codantes de l'adénovirus (« vecteur gutless »), par exemple. Ces vecteurs comportent généralement un acide nucléique hétérologue, dont le transfert dans les cellules ou l'étude est recherché. Cet acide nucléique peut être inséré en différents sites dans le génome recombinant, en remplacement des régions délétées ou en d'autres positions. Des exemples de vecteurs adénoviraux sont décrits notamment dans Levrero et al., Gene 101 (1991) 195; Gosh-Choudhury et al., Gene 50 (1986) 161, WO94/12649, WO94/28152, WO94/28938, WO95/34671, WO96/10088, WO95/02697, WO95/27071, etc., incorporés à la présente par référence.

Les adénovirus recombinants sont produits par transfection de l'ADN du virus recombinant dans une lignée cellulaire d'encapsidation compétente. Il peut s'agir d'une transfection simple, lorsque l'on peut disposer d'une construction portant l'ensemble du génome du virus recombinant, ou, comme c'est le cas le plus souvent, d'une co-transfection de plusieurs fragments d'ADN apportant les différentes parties du génome viral recombinant. Dans ce cas, le procédé implique une ou plusieurs étapes de recombinaison homologue entre les différentes constructions dans la lignée cellulaire d'encapsidation, pour générer l'ADN du virus recombinant. Pour la mise en oeuvre de l'une ou l'autre de ces méthodes, il est donc nécessaire de disposer des constructions appropriées, portant l'ensemble ou des parties du génome de l'adénovirus recombinant que l'on souhaite produire.

Il existe différentes méthodes décrites dans l'art antérieur pour la préparation de ces constructions in vitro. La technique la plus généralement utilisée consiste à isoler l'ADN viral puis à le modifier in vitro par les méthodes classiques de biologie moléculaire (digestion, ligature, etc.). Les constructions obtenues sont ensuite purifiées et utilisées pour transfecter les lignées d'encapsidation. Toutefois, cette technique implique la production de stocks de virus et la purification d'ADN viral pour chaque construction ou pour toute manipulation de l'ADN du virus recombinant, et n'est donc pas adaptée à la production de stocks différents ou de banques. Pour remédier à ces inconvénients, il a été proposé d'utiliser des plasmides procaryotes pour préparer les ADN viraux utilisables pour la transfection. En particulier, Bett et al. (PNAS 91 (1994) 8802) décrit la construction d'un plasmide réplicatif chez E.coli comportant un génome adénoviral modifié (plasmide pBHG10). Plus précisément, ce plasmide porte un génome adénoviral délété des régions E1, E3 et Psi, circularisé par jonction des séquences ITR, et qui comprend, insérée au niveau de la région 188-1339 du génome de l'adénovirus, une partie du plasmide pBR322. Ce plasmide peut être répliqué chez E. Coli, manipulé pour l'insertion de gènes d'intérêt, mais il présente toujours des inconvénients. Son utilisation pour la production de virus implique notamment une linéarisation des ITR jointives et une recombinaison qui, compte tenu des constructions, conduit à l'incorporation, dans le génome adénoviral recombinant, de régions provenant du plasmide procaryote. D'autres plasmides de ce type et présentant le même genre d'inconvénients ont été décrits par exemple par Graham (EMBOJ. 3(12) (1984) 2917). Plus récemment, de nouveaux procédés fondés en particulier sur la recombinaison homologue de deux plasmides ont été décrits, utilisant les chromosomes artificiels de la levure (YACs, Ketner et al. 1994) ou la bactérie (Chartier et al 1996, Crouzet et al 1997, He et al, 1998). Ces méthodes bien que plus performantes que les précédentes sont plus complexes. Le système YAC nécessite le culture et la manipulation de levures (Ketner et al, 1994). Dans les systèmes E.Coli, plusieurs étapes (dont certaines critiques électrotransformation, sélection sucrose) se succèdent. On notera en particulier que dans tous les cas, une ou plusieurs étapes de criblage des clones sont nécessaires pour sélectionner un vecteur recombinant final clonal portant un génome adénoviral infectieux. Ces procédés, s'ils permettent effectivement de produire, de façon clonale, des stocks d'un adénovirus portant un gène thérapeutique donné, ne sont cependant pas utilisables de manière satisfaisante pour la production d'adénovirus recombinants à haut débit, notamment pour la production en simultanée de multiples adénovirus recombinants incorporant des acides nucléiques différents.

Un frein à l'utilisation du vecteur adénoviral comme système de transfert ou d'analyse de gène réside donc dans la complexité et le nombre des opérations permettant de produire ces virus. Il existe donc dans l'art antérieur un besoin clair de pouvoir disposer de plasmides appropriés, facilement manipulables et amplifiables in vitro, pour la préparation de génomes adénoviraux recombinants. Il est également important que les génomes ainsi produits soient pratiquement dépourvus de régions provenant du plasmide, qui sont susceptibles (i) d'induire une réponse immunitaire (ii) de coder pour des protéines de résistance et (iii) de réduire la capacité du virus en tant que vecteur. Il existe également un besoin de méthodologies permettant de générer de manière simplifiée des adénovirus recombinants de façon clonale, rapide et en grand nombre.

La présente invention permet en particulier de remédier à ces inconvénients. La présente invention décrit en effet de nouvelles compositions et méthodes pour la production d'adénovirus recombinants répondant à ces critères. Les compositions et méthodes de l'invention permettent notamment une production clonale rapide, efficace et à haut débit d'adénovirus recombinants utilisables thérapeutiquement ou pour la recherche de cibles pharmaceutiques.

La présente invention repose en particulier sur l'utilisation de deux plasmides (en particulier procaryotes), capables de générer en une étape de recombinaison homologue dans une cellule hôte (en particulier procaryote), un plasmide comprenant un génome adénoviral complet, facilement excisable pour produire des adénovirus recombinants.

D'une manière générale, le procédé de l'invention comprend donc :
Dans une première étape, la construction d'un premier plasmide (appelé « navette »), de préférence procaryote, comprenant un premier génome adénoviral recombinant tronqué comprenant au moins un acide nucléique hétérologue (étape de clonage). Le premier génome tronqué comprend une ITR, un acide nucléique hétérologue, une région d'homologie adénovirale, et éventuellement une séquence d'encapsidation.
Dans une deuxième étape, ce premier plasmide est mis en contact avec un second plasmide (appelé « parent »), comprenant un deuxième génome adénoviral recombinant tronqué, complémentaire du premier, permettant par recombinaison homologue, la production d'un plasmide final comprenant un génome adénoviral recombinant complet (étape de recombinaison). Le deuxième génome adénoviral tronqué comprend au moins une ITR, une région d'homologie adénovirale identique à celle présente dans le premier, et une séquence d'encapsidation (si celle-ci n'est pas présente dans le premier). Ce deuxième génome adénoviral tronqué peut en outre comprendre également un autre acide nucléique d'intérêt.
Dans une troisième étape, le génome adénoviral recombinant complet est excisé du plasmide final et introduit dans une lignée d'encapsidation, pour produire les adénovirus recombinants incorporant le génome adénoviral recombinant complet (étape de production des adénovirus).
Dans une quatrième étape, facultative, les adénovirus recombinants sont utilisés pour infecter :
   - un matériel biologique comprenant des cellules, dans le but d'analyser in vitro les propriétés de l'acide nucléique (étape d'analyse fonctionnelle).
   - Une culture cellulaire in vitro ou ex vivo, dans le but de produire une protéine, polypeptide ou peptide codé par l'acide nucléique hétérologue.

La présente invention permet ainsi d'obtenir, en une étape (recombinaison homologue), un plasmide procaryote portant un génome adénoviral fonctionnel (complet), excisable par une ou des enzymes appropriées. Ce plasmide final résulte ainsi de l'intégration d'un premier plasmide (navette) portant des séquences adénovirales (au minimum une séquence ITR et d'encapsidation) et muni d'un acide nucléique hétérologue d'intérêt, dans un second plasmide (parent) portant des séquences adénovirales complémentaires (au minimum une seconde séquence ITR) par un événement de recombinaison homologue via une séquence commune aux deux plasmides (séquence d'homologie adénovirale). Cet événement de recombinaison homologue donne naissance, via ce co-agrégat, à un génome adénoviral fonctionnel.

Un des avantages du procédé de l'invention réside dans sa simplicité, qui permet sa réalisation en parallèle avec de nombreux plasmides navettes. Ainsi, la première étape du procédé comprend avantageusement le clonage d'une banque d'acides nucléiques dans les plasmides navettes, générant ainsi une banque plasmides finaux porteurs d'un génome adénoviral fonctionnel, ayant une structure identique, à l'exception des inserts qu'ils comprennent. Cette étape de clonage est préférentiellement réalisée en conservant chaque clone individualisé, par exemple dans un puits de plaque de microtitration. En outre, cette étape peut être réalisée de manière automatisée. La banque de plasmides navettes obtenue est ensuite utilisée dans l'étape de recombinaison, chaque plasmide navette de la banque étant mis en contact avec le plasmide parent. Ce procédé conduit ainsi à la production, en parallèle et en simultanée, de multiples adénovirus recombinants comprenant un acide nucléique hétérologue (i.e. une banque d'expression adénovirale). Cette banque peut alors être testée sur le matériel biologique (étape 4), afin d'identifier des clones présentant une activité biologique recherchée.

L'invention peut être utilisée avec tout type d'adénovirus, de cellules procaryotes et à partir de différentes banques d'acides nucléiques, comme il sera illustré en détails dans la suite du texte.

### Définitions

. Adénovirus recombinant : Le terme adénovirus recombinant désigne au sens de l'invention tout adénovirus dont le génome a été modifié, par délétion et/ou insertion et/ou substitution de bases. Il s'agit donc plus particulièrement d'une particule adénovirale, généralement infectieuse, comprenant un génome adénoviral recombinant. Selon la ou les modifications apportées au génome, le virus recombinant peut être défectif pour la réplication, c'est-à-dire incapable de réplication et/ou propagation autonome dans une cellule. L'adénovirus recombinant peut être préparé à partir de tout sérotype d'adénovirus, notamment d'adénovirus humains (par exemple du type C tels que Ad5, Ad2, Ad7, Ad12, etc) ou animaux (tels que des adénovirus canins, comme par exemple CAV-2).
. Génome adénoviral : Le terme « génome adénoviral » désigne la molécule d'ADN présente dans un adénovirus, ou sa séquence, copie ou réplique. Un génome adénoviral recombinant est un acide nucléique dont la séquence correspond à la séquence d'un génome d'adénovirus, et comprend une ou plusieurs modifications. Les modifications comprennent par exemple des délétions (par exemple de tout ou partie des régions E1, E2, E3, E4, etc.), des insertions (comme par exemple d'un ou plusieurs acides nucléiques hétérologues) ou des changements d'usage de codons.

Le génome adénoviral recombinant généré par les compositions et méthodes de l'invention est avantageusement un génome « complet » ou « fonctionnel », c'est-à-dire ne nécessitant pas l'apport d'autres régions par recombinaison ou ligature pour la production des stocks viraux dans les lignées d'encapsidation choisies. Un tel génome « complet » comprend donc avantageusement au moins une séquence d'encapsidation et un acide nucléique hétérologue, flanqués d'une séquence ITR à chaque extrémité. Une autre caractéristique avantageuse des plasmides selon l'invention provient du fait que le génome adénoviral recombinant complet obtenu n'est pas interrompu par des régions du plasmide procaryote. De ce fait, les génomes produits ne contiennent essentiellement pas de régions du plasmide dont les inconvénients ont été mentionnés ci-avant. Par ailleurs, dans les plasmides selon l'invention, les ITR du génome adénoviral ne sont pas jointives, ce qui permet d'obtenir des génomes viraux recombinants complets linéaires, directement utilisables pour produire les virus recombinants.

Le génome adénoviral recombinant comprend au moins des séquences ITR et une séquence permettant l'encapsidation. Les séquences inversées répétées (ITR) constituent l'origine de réplication des adénovirus. Elles sont localisées aux extrémités du génome viral , d'où elles peuvent être isolées aisément selon les techniques classiques de biologie moléculaire connues de l'homme du métier. La séquence nucléotidique des séquences ITR des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence ITR gauche correspond à la région comprenant les nucléotides 1 à 103 du génome.

La séquence d'encapsidation (également désignée séquence Psi) est nécessaire à l'encapsidation du génome viral. Elle est localisée dans le génome des adénovirus sauvages, entre l'ITR gauche et la région E1. Elle peut être isolée ou synthétisée artificiellement par les techniques classiques de biologie moléculaire. La séquence nucléotidiques de la séquence d'encapsidation des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, une séquence d'encapsidation fonctionnelle est comprise entre les nucléotides 194 et 358 du génome.

Dans un mode de réalisation préféré de l'invention, le génome de l'adénovirus est dépourvu de tout ou partie de la région E1. La région E1 est en effet essentielle à la réplication virale et son inactivation conduit à la formation de virus défectifs pour la réplication, c'est-à-dire incapables de se répliquer de façon autonome après transfert génique in vivo. La région E1, ou tout autre région virale considérée, peut être rendue non fonctionnelle par toute technique connue de l'homme du métier, et notamment par suppression totale, substitution, délétion partielle, ou addition d'une ou plusieurs bases dans le ou les gènes considérés. De telles modifications peuvent être facilement réalisées directement sur les plasmides de l'invention, par exemple, au moyens des techniques du génie génétique. Avantageusement, le génome de l'adénovirus généré est dépourvu d'une partie de la région E1 comprise entre les nucléotides 454 à 3328 (fragment PvuII-BgIII) ou 382 à 3446 (fragment HinfII-Sau3A).

Un génome adénoviral recombinant tronqué désigne un ADN correspondant à la séquence d'une partie terminale d'un génome adénoviral, c'est-à-dire depuis une extrémité (ITR). Deux génomes recombinants tronqués sont dits complémentaires lorsqu'ils portent chacun une partie complémentaire d'un génome adénoviral, et qu'ils peuvent reconstituer, par recombinaison homologue, un génome adénoviral recombinant complet.
. Acide nucléique hétérologue : Le terme « acide nucléique hétérologue » désigne tout acide nucléique inséré dans le génome adénoviral recombinant, dont le transfert, l'expression ou l'étude fonctionnelle sont recherchés. Il s'agit essentiellement d'un acide nucléique ayant une origine différente d'un adénovirus (« hétérologue »), par exemple provenant d'une cellules humaine, animale, végétale, virale (autre que l'adénovirus utilisé comme vecteur), procaryote, eucaryote inférieure, ou d'origine synthétique ou semi-synthétique. La taille de l'acide nucléique hétérologue peut varier, dans la mesure où le génome adénoviral recombinant le contenant n'excède pas la taille maximale permettant son encapsidation dans une particule adénovirale (au total, moins de 40 kb). Ainsi, il peut s'agir d'un acide nucléique d'une longueur supérieure à 30kb, lorsque suffisamment de régions de l'adénovirus ont été délétées. L'acide nucléique peut à cet égard comprendre une région codant pour une protéine, un polypeptide ou un peptide donnés, par exemple un ADNc, un ADNg ou un ADN synthétique. Il peut également s'agir d'un acide nucléique de structure inconnue, provenant par exemple d'un clone d'une banque d'acides nucléiques. Par ailleurs, le génome adénoviral recombinant peut comprendre plusieurs acides nucléiques hétérologues, insérés en des sites différents du génome.

### Description des plasmides

Comme indiqué ci-avant, la présente invention fait appel à deux plasmides (procaryotes), le plasmide navette et le plasmide parent, comprenant chacun un fragment complémentaire d'un génome d'adénovirus recombinant. L'un de ces plasmides au moins possède un acide nucléique hétérologue (ou un site d'insertion d'un tel acide nucléique), d'intérêt pour la thérapie génique, la production d'une protéine recombinante ou pour une approche de génomique fonctionnelle, par exemple. Les extrémités (séquences ITR) de chacun des génomes tronqués portées par chacun des plasmides sont bordées (en 5' pour l'ITR gauche et en 3' pour l'ITR droit) par un site non présent dans ledit génome, pour permettre l'excision du génome complet, après recombinaison. Ces plasmides portent un génome tronqué de l'adénovirus et ne peuvent générer individuellement un génome adénoviral infectieux. Ces deux plasmides possèdent chacun la partie complémentaire à l'autre pour générer, par co-intégration, un plasmide final possédant alors l'ensemble d'un génome d'adénovirus bordé par deux ITR et par au moins un site de restriction non présent dans ledit génome. Le fragment de génome d'adénovirus recombinant présent dans ces plasmides est un génome incomplet qui ne peut par lui même s'avérer infectieux par la suite. Ceci est particulièrement intéressant puisque seul le co-intégrat des deux plasmides est capable de générer un génome fonctionnel.

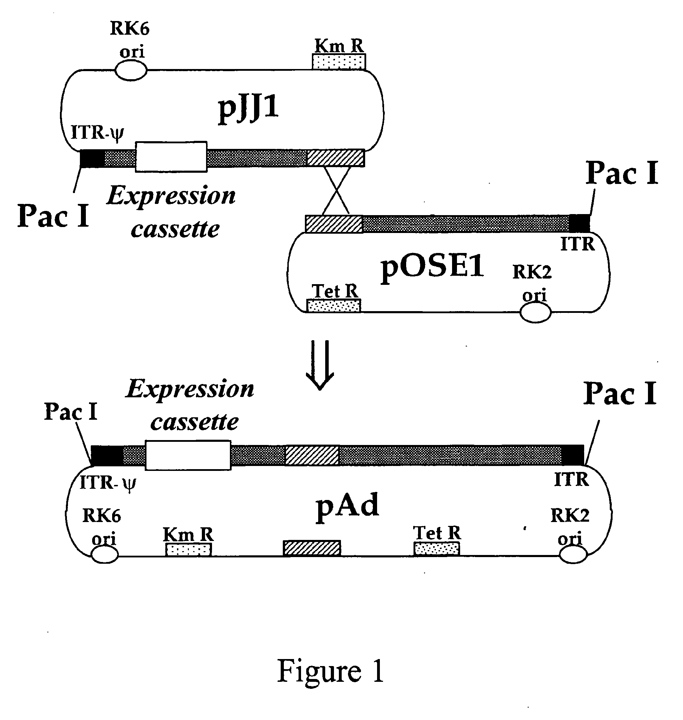

De tels plasmides sont représentés par exemple sur la figure 1 et sont décrits plus en détails dans la suite du texte. Ces plasmides sont préférentiellement des plasmides procaryotes, et comprennent généralement une région plasmidique et une région adénovirale. La région plasmidique permet la réplication et/ou la sélection du plasmide dans une cellule hôte, en particulier dans une cellule hôte procaryote. La région adénovirale (génome tronqué) apporte une partie du génome adénoviral recombinant qui, après recombinaison avec la région adénovirale du plasmide complémentaire (parent ou navette), permet de reconstituer le génome adénoviral recombinant complet.

La région permettant la réplication dans les cellules procaryotes utilisée dans les plasmides de l'invention peut être toute origine de réplication fonctionnelle dans les cellules choisies. II peut s'agir d'une origine de réplication issue d'un plasmide du groupe d'incompatibilité P (exemple = pRK290) qui permet la réplication dans les souches d'E. Coli pol A. Plus généralement, il peut s'agir de toute origine de réplication issue d'un plasmide se répliquant dans les cellules procaryotes. Ce plasmide peut être un dérivé de pBR322 (Bolivar et al., 1977), un dérivé de puc (Viera et Messing, 1982), ou d'autres plasmides dérivant du même groupe d'incompatibilité, c'est à dire de ColE1 ou de pMB1 par exemple. Ces plasmides peuvent être choisis par ailleurs dans d'autres groupes d'incompatibilité se répliquant chez Escherichia coli. Il peut s'agir de plasmides dérivés de plasmides appartenant aux groupes d'incompatibilité A, B, FI, FII, FIII, FIV, H1, H11, I1, 12, J, K, L, N, OF, P, Q, T, U, W, X, Y, Z ou 9 par exemple. D'autres plasmides peuvent encore être utilisés, parmi lesquels des plasmides ne se répliquant pas chez E. coli mais chez d'autres hôtes tels que B. subtilis, Streptomyces, P. putida, P. aeruginosa, Rhizobium meliloti, Agrobacterium tumefaciens, Staphylococcus aureus, Streptomyces pristinaespiralis, Enterococcus faecium ou Clostridium. A titre préférentiel, on utilise les origines de réplication issues de plasmides se répliquant chez E.coli.

La région permettant la sélection des cellules procaryotes contenant les plasmides de l'invention peut être constituée notamment par tout gène conférant la résistance à un produit, et notamment à un antibiotique. Ainsi, on peut citer les gènes conférant une résistance à la kanamycine (Kan^{r}), à l'ampicilline (Amp,^{r}), à la tétracycline (tet^{r}) ou à la spectinomycine, par exemple, qui sont couramment utilisés en biologie moléculaire (Maniatis et al., 1989). La sélection de plasmides peut se faire par d'autres gènes que des gènes codant pour des marqueurs de résistance à un antibiotique. D'une manière générale, il s'agit d'un gène qui donne à la bactérie une fonction qu'elle ne possède plus (cela peut correspondre à un gène qui a été délété sur le chromosome ou rendu inactif), le gène sur le plasmide rétablissant cette fonction. A titre d'exemple il peut s'agir d'un gène d'un ARN de transfert qui rétablit une fonction chromosomique déficiente (Somoes et al., 1991).

Le plasmide navette et le plasmide parent portent une origine de réplication et un marqueur différents, pour permettre la sélection de chaque élément.

La région adénovirale comprise dans chacun des plasmides correspond essentiellement à la séquence d'un génome adénoviral tronqué. Ces génomes tronqués, portés par chacun des plasmides, sont complémentaires, c'est-à-dire capables de générer un génome adénoviral recombinant complet (et linéaire) après recombinaison homologue. En outre, les génomes tronqués, portés par chacun des plasmides sont préférentiellement bordés par un ou plusieurs sites de restriction non présent dans un génome adénoviral, de sorte que le génome adénoviral recombinant complet produit par recombinaison soit bordé par de tels sites.

### Génome tronqué présent dans le plasmide navette

Le plasmide navette, comme indiqué ci-avant, est destiné à recevoir l'acide nucléique d'intérêt, en vue de son incorporation dans un génome adénoviral recombinant.

La région adénovirale du plasmide navette correspond à la partie gauche ou la partie droite d'un génome adénoviral, depuis son extrémité (une séquence ITR), jusqu'à une région d'homologie adénovirale choisie, qui permettra la recombinaison homologue entre les deux plasmides. En outre ce génome adénoviral comprend une ou plusieurs modifications génétiques.

Dans un premier mode de réalisation, le génome tronqué présent dans le plasmide navette correspond à la partie gauche du génome adénoviral recombinant final. Dans ce mode de réalisation, il comprend par exemple une séquence allant de l'ITR gauche jusqu'à la région codant pour la protéine pIX (et/ou Iva2), l'acide nucléique hétérologue étant inséré en substitution de tout ou partie de la région E1. Dans ce mode particulier de réalisation, la région d'homologie adénovirale est constituée par la région codant pour la protéine pIX (et/ou Iva2).

Dans un autre mode de réalisation, le génome tronqué présent dans le plasmide navette correspond à la partie droite du génome adénoviral recombinant final. Dans ce mode de mise en oeuvre, il comprend par exemple la séquence allant de l'ITR droit jusqu'à la région codant pour la protéine pIX (et/ou Iva2), l'acide nucléique hétérologue étant inséré en substitution de tout ou partie de la région E4 ou E3 par exemple. Dans ce mode particulier de réalisation, la région d'homologie adénovirale est également constituée par la région codant pour la protéine pIX (et/ou Iva2).

Par ailleurs, dans un mode particulier de mise en oeuvre, la région d'homologie est constituée par une séquence adénovirale modifiée. Ainsi, la région d'homologie peut être constituée par exemple par une région de l'adénovirus modifiée par changement de l'usage des codons, en utilisant la dégénérescence du code génétique. De telles modifications ont été décrite dans la demande WO99/25861, incorporée à la présente par référence. Dans un mode particulier de réalisation, la région d'homologie adénovirale est constituée par une région codant pour la protéine pIX (et/ou Iva2) dégénérée.

Il est entendu que, selon la structure choisie du génome adénoviral recombinant final (généré après recombinaison), la région d'homologie adénovirale peut correspondre à différentes régions du génome. En effet, la région adénovirale du plasmide parent peut s'étendre depuis l'ITR gauche jusqu'à la région E3, et c'est alors cette région qui constitue la zone d'homologie adénovirale. Plus généralement, la région d'homologie adénovirale est une séquence correspondant à toute région d'un génome adénoviral sauvage ou modifié, qui permet la recombinaison entre le plasmide navette et le plasmide parent, de manière à générer le génome adénoviral recombinant final. Cette région d'homologie est donc essentiellement identique dans le plasmide navette et dans le plasmide parent. Elle peut correspondre à différentes parties du génome d'un adénovirus, selon le type de constructions utilisé.

Un mode de réalisation particulier de l'invention réside dans un plasmide navette comprenant un génome tronqué comprenant la région ITR gauche, la séquence d'encapsidation, l'acide nucléique hétérologue et une région d'homologie adénovirale constituée par une région codant pour la protéine pIX (et/ou Iva2) sauvage ou dégénérée (cf. plasmides pJJ1 et pIG5, par exemple).

Par ailleurs, dans un mode préféré de l'invention, le génome adénoviral tronqué est bordé, du coté de l'ITR, d'un site non présent dans le génome adénoviral, par exemple Pacl.

### Génome tronqué présent dans le plasmide parent

Le plasmide parent, comme indiqué ci-avant, porte un génome adénoviral tronqué, capable de compléter, par recombinaison, le génome tronqué présent dans le plasmide navette. Sa structure est donc dépendante de la structure du plasmide navette. Par ailleurs, le plasmide parent peut également contenir un acide nucléique, différent de celui porté par le plasmide navette.

Ainsi, lorsque la région adénovirale du plasmide navette correspond à la partie gauche d'un génome adénoviral, le génome tronqué présent dans le plasmide parent correspond à sa partie droite, s'étendant depuis l'ITR jusqu'à la région d'homologie adénovirale. Réciproquement, lorsque la région adénovirale du plasmide navette correspond à la partie droite d'un génome adénoviral, le génome tronqué présent dans le plasmide parent correspond à sa partie gauche, s'étendant depuis l'ITR jusqu'à la région d'homologie adénovirale.

Dans un premier mode de réalisation, le génome tronqué présent dans le plasmide parent correspond à la partie droite du génome adénoviral recombinant final. Dans ce mode de mise en oeuvre, il comprend par exemple la séquence allant de l'ITR droit jusqu'à la région codant pour la protéine pIX (et/ou Iva2). Par ailleurs, le génome peut comprendre des modifications génétiques, telles que des délétions de tout ou partie des régions E4 ou E3 par exemple.

Dans un autre mode de réalisation, le génome tronqué présent dans le plasmide parent correspond à la partie gauche du génome adénoviral recombinant final. Dans ce mode de réalisation, il comprend par exemple une séquence allant de l'ITR gauche jusqu'à une séquence proche de la région E4. Par ailleurs, le génome peut comprendre des modifications génétiques, telles que des délétions de tout ou partie de tout ou partie de la région E1 par exemple.

Préférentiellement, le plasmide parent porte un génome tronqué correspondant à la partie droite du génome adénoviral, et comporte une région E3 non-fonctionnelle. Plus préférentiellement, il comporte une région E4 non fonctionnelle. Encore plus préférentiellement, il comporte une délétion de tout ou partie des phases ORF3 et/ou ORF6 de la région E4. Dans un autre mode de réalisation particulier, le plasmide parent porte un génome tronqué correspondant à la partie droite du génome adénoviral, et comporte des régions E3 et E4 fonctionnelles.

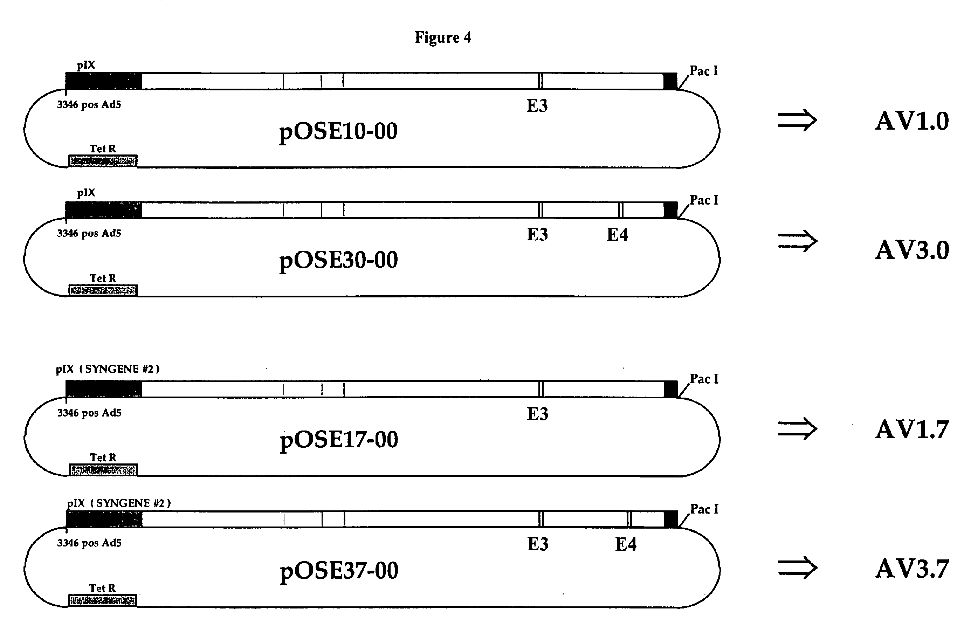

Des exemples particuliers de tels plasmides sont représenté par pOSE1, pOSE10-00, pOSE30-00, pOSE17-00 et pOSE37-00 (cf. Figures 1 et 4).

Dans ce mode de mise en oeuvre, le plasmide navette porte un génome tronqué correspondant à la partie gauche du génome adénoviral, et comporte une délétion de tout ou partie de la région E1.

Deux plasmides particulièrement préférés au sens de l'invention sont le plasmide pOSE17-00 et le plasmide pIG5. pOSE17-00 (plasmide parent) comprend l'origine de réplication du plasmide RK2, le gène de résistance à la tétracycline et possède un fragment du génome adénoviral commençant à la base 3520 de l'Ad5 jusqu'à l'ITR droit qui est bordé par un site Pacl. Ce fragment de génome possède une région E3 non fonctionnelle et une séquence modifiée en pIX et Iva2 comme décrit dans la demande WO99/25861. Le plasmide plG5 (plasmide navette) possède une origine de réplication RK6 et ne peut se répliquer dans des souches E. coli pir-. pIG5 possède les régions ITR et d'encapsidation précédées d'un site Pacl, la cassette d'expression désirée et une région homologue à pOSE17-00 (une séquence modifiée en pIX et Iva2 comme décrit dans la demande WO99/25861). Le génome désiré est ainsi reconstruit par recombinaison homologue grâce à la présence de la région homologue entre les deux plasmides (i.e. la séquence modifiée en pIX et Iva2 comme décrit dans la demande WO99/25861).

Le procédé de l'invention permet ainsi de générer un génome recombinant complet (infectieux) à partir de deux plasmides apportant chacun une partie du génome (un plasmide portant la séquence ITR gauche précédée d'un site Pacl et un ensemble de séquences virales ou non virales et un plasmide portant, précédé de séquences virales ou non virales, l'ITR droit suivi d'un site Pacl, le choix des séquences virales ou non virales dépendent du choix de la construction planifiée, vecteur adénovirus recombinant délété pour tout ou partie des gènes viraux); les deux plasmides présentant des séquences communes suffisantes à la réalisation de la recombinaison homologue et présentant un chevauchement dans une séquence permettant l'événement de recombinaison homologue. En particulier, la méthode permet dans un schéma comparable à celui présenté pour l'introduction de séquences exogènes dans la région E1, d'introduire des séquences exogènes dans la région E4 à l'aide des plasmides appropriés. Dans ce cas, la recombinaison à lieu entre un plasmide parental (possédant les régions ITR gauche et d'encapsidation précédé d'un site de restriction pour l'enzyme Pacl et d'un génome adénoviral tronqué à proximité de la région E4) et un plasmide navette (possédant une séquence proche de la région E4 identique au vecteur parental -qui sera impliquée dans l'évènement de recombinaison- suivi de la cassette d'expression de la séquence d'intérêt, puis de la région ITR droit et d'un site Pacl unique).

Selon un mode de réalisation particulièrement avantageux, le génome de l'adénovirus produit est également dépourvu de tout ou partie de la région E3 et/ou E4 et/ou IVA2. Ces délétions supplémentaires permettent d'accroître la sécurité du vecteur et d'augmenter sa capacité. Préférentiellement, le génome adénoviral est dépourvu d'une partie de la région E4 comprenant au moins les phases ORF3 et ORF6. Le génome adénoviral peut également être modifié comme décrit dans la demande FR9413355, incorporée à la présente par référence, de manière à éviter les risques de contamination par des particules de réplication. Dans un mode particulier, le génome recombinant adénoviral complet obtenu est un génome dit « gutless », c'est-à-dire dépourvu de toute région codante de l'adénovirus. Dans ce mode de réalisation, le génome tronqué des plasmides comporte d'une part, une ITR et la région d'encapsidation et d'autre part, une ITR, la région d'homologie pouvant être constituée par l'acide nucléique hétérologue lui-même ou par une séquence artificielle, incluse dans chaque plasmide.

A cet égard, la région d'homologie présente dans chaque plasmide peut, d'une manière générale, être une séquence non virale, artificielle ou non, permettant la recombinaison homologue.

Le génome recombinant peut également comporter des cassettes excisables in vivo (WO97/47757) ou des séquences génétiques modifiées (gènes de la fibre ou du penton) permettant de modifier le tropisme du virus. En outre, l'organisation génomique du virus peut également être modifiée pour améliorer la sécurité des virus produits (WO96/13596).

Comme indiqué ci-avant, le génome d'adénovirus recombinant est avantageusement bordé d'un ou plusieurs sites de restriction absents dudit génome. Ce ou ces sites permettent d'exciser de manière simple et efficace le génome adénoviral recombinant du plasmide. La séquence génomique de l'adénovirus étant connue et accessible, l'homme du métier peut sélectionner par des expériences de routine des sites de restriction absent de ce génome. A titre d'exemple, on peut citer les sites PacI, NspV et SwaI (pour Ad5) ou Snabl (dans Ad2). Il est également possible de rendre certains sites uniques en modifiant la séquence du génome adénoviral. Ainsi, d'autres enzymes peuvent être utilisées si les sites de restriction correspondants sont supprimés modifiés ou délétés dans la séquence adénovirale construite chez E. coli. Les sites peuvent être positionnés directement à coté des extrémités du génome adénoviral, ou espacés de quelques paires de bases.

Les plasmides selon l'invention peuvent être construits en utilisant des adénovirus d'origine diverse. Différents sérotypes d'adénovirus, dont la structure et les propriétés varient quelque peu, ont en effet été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO 94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. Selon un mode particulier de réalisation de l'invention, l'adénovirus utilisé est un adénovirus d'origine humaine. Selon un autre mode avantageux, l'adénovirus est un adénovirus d'origine animale.

### Recombinaison homologue et production des virus

L'étape de recombinaison homologue (permettant la reconstruction du génome complet) peut être réalisée selon les techniques connues de l'homme du métier, par transfection (ou co-transfection) des plasmides dans une cellule appropriée, de préférence une cellule procaryote. Dans un mode particulier de réalisation, la recombinaison homologue est effectuée chez E. coli en utilisant une souche polA afin de sélectionner les événements de recombinaison homologue. Il est évident que ces constructions peuvent aussi être réalisées en l'absence de systèmes permettant de sélectionner des événements de recombinaison. En effet de tels événements de recombinaison peuvent être criblés par minipréparation, perte ou acquisition d'un marqueur, ou bien criblage à l'aide de sondes radioactives spécifiques pour les jonctions obtenues ou perdues. De plus, il existe d'autres techniques permettant de sélectionner des événements de recombinaison homologue chez E. coli. Parmi celles -ci, citons l'utilisation de plasmides thermosensibles pour leur réplication (Hamilton et al., 1989), l'utilisation de molécules circulaires non réplicatives (décrite par exemple par Slater et Maurer, 1993), l'utilisation de souches dans lesquelles le vecteur utilisé ne se réplique pas (Miller et Mekalanos, 1988, Zeef et al., 1994), etc. Tous ces systèmes peuvent être utilisés à la place de souches polA et d'une transformation avec un plasmide dérivé de pBR322, ou ses nombreux dérivés, ou d'autres plasmides à réplication PolA-dépendante ou bien des plasmides ne se réplicant pas chez Escherichia coli.

Un autre objet de la présente demande concerne toute cellule procaryote contenant un plasmide tel que défini ci-avant. Il peut s'agir en particulier de toute bactérie pour laquelle il existe un système de vecteur où l'ADN recombinant peut être introduit. Citons par exemple Escherichia coli, Salmonella typhimurium, Bacillus subtilis, Pseudomonas putida, Pseudomonas aeruginosa, Agrobacterium tumefaciens, Rhizobium meliloti ou les bactéries du genre Streptomyces. Ces cellules sont obtenues avantageusement par transformation selon les techniques connues de l'homme du métier. La transformation peut notamment être effectuée par la technique de transformation au CaCl₂ (Dagert et Ehrlich, 1979), ou celle mise au point par Hanahan et al. (1983) ou toute technique dérivée de celle-ci (Maniatis et al., 1989), ainsi que par électrotransformation (Wirth et al., 1989). Voir également les techniques générales de Biologie Moléculaire ci-après.

Un autre objet de la présente invention réside dans un procédé de production de génomes d'adénovirus recombinants. Selon ce procédé, des cellules procaryotes telles que décrites ci-dessus sont cultivées puis, dans une deuxième étape, les plasmides sont récupérés. Avantageusement, la culture est réalisée pendant un temps suffisamment long pour produire des quantités appropriées de plasmide. Le plasmide peut être récupéré par toute technique connue de l'homme du métier pour la préparation d'ADN plasmidique. Ainsi, il peut être récupéré par préparation d'un lysat clair suivi d'une centrifugation dans un gradient en chlorure de césium (Maniatis et al., 1989). D'autres techniques peuvent être utilisées, faisant appel à d'autres méthodes de lyse utilisant le triton X-100, par exemple (Ausubel et al., 1987), ou bien une colonne d'échange d'anions après l'étape de lyse et de séparation de l'ADN plasmidique vis à vis de la majorité de l'ADN chromosomique et des protéines. Les plasmides ainsi récupérés peuvent ensuite être purifiés et traités en présence de l'enzyme de restriction correspondant aux sites bordant le génome viral. Ceci permet en une seule étape de générer un génome d'adénovirus recombinant linéaire, directement utilisable pour la production clonale de virus recombinants.

A cet égard, une première méthode pour préparer les virus recombinants consiste à transfecter le génome viral produit à partir des plasmides de l'invention dans une lignée cellulaire d'encapsidation compétente, c'est-à-dire portant en trans toutes les fonctions nécessaires à la complémentation du virus défectif. Ces fonctions sont préférentiellement intégrées dans le génome de la cellule, ce qui réduit les risques de recombinaison, et confère une stabilité accrue à la lignée cellulaire.

Une seconde approche consiste à co-transfecter dans une lignée cellulaire appropriée le génome recombinant préparé et l'ADN d'un ou de plusieurs virus ou plasmide helper. Selon cette méthode, il n'est pas nécessaire de disposer d'une lignée cellulaire compétente capable de complémenter toutes les fonctions défectives de l'adénovirus recombinant. Une partie de ces fonctions est en effet complémentée par le ou les virus helper. Ce ou ces virus helper sont eux-mêmes défectifs.

Parmi les lignées cellulaires utilisables, on peut citer notamment la lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %). La transfection peut être réalisée avantageusement directement avec le produit de digestion du plasmide obtenu selon le procédé décrit ci-avant, sans étape de purification du génome adénoviral. D'autres lignées sont par exemple des lignées produites à partir de cellules embryonnaire de rétine (HER), de cellules de foie, etc. Il peut s'agir de lignées complémentant les fonctions E1 (293, cellules PERC-6), E1 et E4 (IGRP2), E1 et E2, etc. De telles lignées ont été décrite dans l'art antérieur et sont utilisables par l'homme du métier.

Les adénovirus ainsi produits peuvent être isolés ou purifiés par les techniques connues de l'homme du métier (chlorure de césium, chromatographie, etc.). Ils peuvent être utilisés dans différentes applications, comme la production de produits thérapeutiques ou prophylactiques in vitro, ex vivo ou in vivo, ou également pour l'analyse fonctionnelle du génome (et la constitution de banques).

Les bénéfices de l'utilisation du nouveau procédé sont les suivants: Une simplification et une plus grande rapidité dans la génération du recombinant, et la non nécessité d'une étape de criblage liée aux systèmes de sélection multiples imposés au procédé et à la structure d'un vecteur parental non fonctionnel.

Contrairement aux procédés connus décrits, la robustesse du procédé est basée sur la simultanéité des pressions de sélections qui conduit en une seule étape à la sélection du seul plasmide final portant le génome adénoviral fonctionnel. La génération d'un nouveau vecteur recombinant en une seule étape réduit considérablement le temps de travail nécessaire en comparaison avec les procédés connus. Toute étape de criblage étant inutile, la quantité de travail nécessaire est considérablement allégée en comparaison avec les procédés connus. De plus, la non-nécessité de criblage ouvre la voie à la génération simultané de nombreux vecteurs adénoviraux recombinants par un même intervenant.

Un autre avantage du procédé d'invention réside dans l'aspect non viable des deux plasmides recrutés pour la génération du plasmide final portant le génome fonctionnel. Aucun des deux plasmides initiaux ne possède séparément l'ensemble des fonctions lui permettant de générer un génome adénoviral fonctionnel. Aussi, dans le cas extrême d'une fuite dans les sélections imposées (antibiotiques), si deux types de constructions (la construction souhaitée et les plasmides initiaux) devaient co-exister après la recombinaison dans la souche bactérienne, ce résultat n'aurait pas d'incidence sur la génération du vecteur recombinant souhaité, une fois ce mélange transfecté dans la cellule eucaryote productrice. En effet, seule la construction souhaitée résultant de la recombinaison homologue est viable et s'amplifie dans la cellule eucaryote productrice, les plasmides initiaux étant non-viables (absence d'au moins une région ITR pour chaque plasmide).

Les caractéristiques citées plus haut du procédé d'invention concourent à une simplification de la construction de vecteurs adénoviraux et ouvrent la possibilité de génération à au débit de vecteurs recombinants. Il peut s'agir de construire en simultanée des vecteurs adénoviraux recombinants portant des séquences connues dont on veut valider la fonction, ou d'un ensemble de vecteurs recombinants portant des séquences de nature inconnue dont on veut découvrir la fonction. Dans ce dernier cas on parle de banques d'expression adénovirale.

### Dans une application de thérapie génique

Les adénovirus peuvent être utilisés dans des applications thérapeutiques. Dans ce but, l'acide nucléique hétérologue peut comporter un ou plusieurs gènes thérapeutiques et/ou un ou plusieurs gènes codant pour des peptides antigéniques.

Les gènes thérapeutiques qui peuvent ainsi être transférés sont tout gène dont la transcription et éventuellement la traduction dans la cellule cible génèrent des produits ayant un effet thérapeutique. Il peut s'agir d'un produit homologue vis-à-vis de la cellule cible (c'est-à-dire un produit qui est normalement exprimé dans la cellule cible lorsque celle-ci ne présente aucune pathologie). Dans ce cas, l'expression permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit peut également être hétérologue vis-à-vis de la cellule cible. Dans ce cas, une protéine exprimée peut par exemple compléter ou apporter une activité déficiente dans la cellule lui permettant de lutter contre une pathologie.

Parmi les produits thérapeutiques, on peut citer plus particulièrement les enzymes, les dérivés sanguins, les hormones, les lymphokines : interleukines, interférons, TNF, etc (WO93/19191), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc; les apolipoprotéines : ApoAI, ApoAIV, ApoE, etc (WO94/25073), la dystrophine ou une minidystrophine (FR 9111947), les gènes suppresseurs de tumeurs : p53, Rb, Rap1 A, DCC, k-rev, etc (WO9424297), les gènes codant pour des facteurs impliqués dans la coagulation : Facteurs VII, VIII, IX, les gènes suicide (TK, etc), etc.

Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Comme indiqué plus haut, l'acide nucléique d'intérêt peut également comporter un ou plusieurs gènes codant pour un peptide antigénique, capable de générer chez l'homme une réponse immunitaire. Dans ce mode particulier de mise en oeuvre, l'invention permet donc la réalisation de vaccins permettant d'immuniser l'homme, notamment contre des microorganismes ou des virus. Il peut s'agir notamment de peptides antigéniques spécifiques du virus d'Epstein barr, du virus HIV, du virus de l'hépatite B (EP 185 573), du virus de la pseudo-rage, ou encore spécifiques de tumeurs (EP 259 212).

Généralement, l'acide nucléique d'intérêt comprend également des séquences permettant l'expression du gène thérapeutique et/ou du gène codant pour le peptide antigénique dans la cellule infectée. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs des gènes E1A, MLP (Major Late Promoter), CMV (Cytomégalovirus), RSV (Rous Sarcoma Virus), etc. En outre, ces séquences d'expression peuvent être modifiées par addition de séquences d'activation, de régulation, etc. Préférentiellement, on pourra utiliser des promoteurs régulable par la tétracycine du type Tet on/off ou off/on, ou inductible par ecdysone ou dexaméthasone. Par ailleurs, lorsque le gène inséré ne comporte pas de séquences d'expression, il peut être inséré dans le génome du virus défectif en aval d'une telle séquence. Enfin, l'acide nucléique d'intérêt peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

La présente invention concerne également toute composition pharmaceutique comprenant un ou plusieurs adénovirus recombinants préparé selon ce procédé. Les compositions pharmaceutiques de l'invention peuvent être formulées en vue d'une administration par voie topique, orale, parentérale, intranasale, intraveineuse, intramusculaire, sous-cutanée, intraoculaire, transdermique, etc.

Préférentiellement, la composition pharmaceutique contient des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution de virus, et est déterminé par infection d'une culture cellulaire appropriée, et mesure, généralement après 15 jours, du nombre de plages de cellules infectées. Les techniques de détermination du titre pfu d'une solution virale sont bien documentées dans la littérature.

Selon la séquence d'ADN hétérologue insérée, les adénovirus de l'invention peuvent être utilisés pour le traitement ou la prévention de nombreuses pathologies, incluant les maladies génétiques (dystrophie, fibrose cystique, etc), les maladies neurodégénératives (alzheimer, parkinson, ALS, etc), les cancers, les pathologies liées aux désordres de la coagulation ou aux dyslipoprotéinémies, les pathologies liées aux infections virales (hépatites, SIDA, etc), etc.

### Dans une application de production de protéines recombinantes.

Le procédé d'invention permet de générer des vecteurs adénoviraux qui peuvent permettre la production de protéines recombinantes, en particulier dans des cellules humaines en culture . Il peut s'agir de protéines telles que l'albumine sérique humaine, les interférons humains, des anticorps humains, de l'insuline humaine, de facteurs hématopoïétiques, de facteurs tels que le facteur IX ou VII, de facteurs thrombolytiques tel que l'activateur plasminogène tissulaire, divers facteurs de croissance, facteurs trophiques, cytokines, de peptides du système nerveux central, d'opioides, d'enzymes, d'antigènes viraux mais aussi de protéines d'autres organismes comme celle de plantes.

### Dans une application de génomique fonctionnelle:

A l'aide des mêmes outils de fabrication cités plus haut, le procédé est exploité dans une application de validation ou de découverte de nouvelles cibles dans le sens compris par l'industrie pharmaceutique.

La présente invention concerne également toute exploitation d'une composition utilisant la méthode décrite. En particulier, en aval, toute préparation permettant l'obtention et l'intégration d' acide nucléique quelque soit leur nature dans un premier plasmide navette au sens de l'invention ainsi que l'utilisation desdits plasmides pour générer un ou de multiples adénovirus recombinants par les méthodes diverses d'automatisation. Ces adénovirus recombinants peuvent alors être utilisés dans de multiples modèles in vitro et in vivo pour évaluer la fonctionnalité des séquences qu'ils portent. La fonctionnalité des séquences portées par les adénovirus recombinants générés peut-être évaluée par l'un des nombreux tests fonctionnels disponibles à l'homme du métier.

Un objet de l'invention réside donc également dans un procédé de préparation de banques d'expression adénovirales, comprenant les étapes suivantes :
- la construction d'une banque primaire dans un vecteur navette tel que décrit ci-avant,
- la transformation d'une culture bactérienne avec ladite banque primaire, en présence d'un plasmide parent selon l'invention,
- l'introduction des plasmides obtenus, ou des génomes adénoviraux complets après excision par digestion enzymatique, dans une cellule productrice d'adénovirus.
- Eventuellement, l'infection d'un matériel biologique comprenant des cellules afin d'analyser les clones de la banque.

Ce procédé comprend donc une première étape de préparation d'une banque primaire d'acides nucléiques dans le plasmide navette de l'invention avant d'appliquer l'invention et d'utiliser la banque adénovirale dans un modèle expérimental. La constitution d'une telle banque primaire dans le plasmide de l'invention (plasmide navette) peut être réalisée par les méthodes de biologie moléculaire connues de l'homme du métier. En particulier, la banque primaire peut être construite par la succession des opération suivantes: a) obtention d'ARNm (ou d'ARN totaux) à partir d'une population cellulaire, b) préparation des ADN complémentaires de la population d'ARNm isolée c) éventuellement la préparation d'une banque d'acides nucléiques dans laquelle les ADNc sont placés sous le contrôle d'un promoteur fort, ou plus particulièrement sous le contrôle d'un promoteur régulable, d) incorporation des ADNc (ou de la cassette d'expression) dans le plasmide navette de l'invention.

La présente invention a donc également pour objet une banque d'acides nucléiques clonée dans un vecteur ou plasmide navette tel que décrit précédemment.

Dans la deuxième étape, la co-transformation de la banque primaire de plasmides navettes avec un plasmide parental décrit dans l'invention permet l'obtention d'une banque de plasmides portant des génomes adénoviraux fonctionnels. Cette étape peut être réalisée soit par co-transformation simultanée des plasmides dans les bactéries, soit par transfections séquentielles. Ainsi, le plasmide parent peut être, dans un premier temps, introduit dans les bactéries, puis les plasmides navettes sont transfectés dans un deuxième temps.

Dans la troisième étape, les plasmides (ou les génomes recombinants fonctionnels excisés sont transfectés dans une cellule transcomplémentante, ce qui permet de générer une banque d'adénovirus recombinants. Préférentiellement, les plasmide obtenus sont isolés et/ou purifiés, et les génomes recombinants sont excisés par traitement avec l'enzyme (ou les enzymes) appropriés, ne coupant pas le génome adénoviral.

Dans la quatrième étape, l'infection d'une population cellulaire choisie (ou d'un matériel biologique) à l'aide de la banque d'adénovirus recombinants permet d'analyser les propriétés biologiques ou fonctionnelles des clones de la banque. Pour cela, la population cellulaire choisie et infectée peut être placée dans des conditions permettant l'expression de l'acide nucléique et ainsi d'identifier un phénotype recherché par l'étude de la population cellulaire. Il est ensuite possible de remonter au vecteur inducteur et ainsi de caractériser l'ADN conférant le phénotype recherché.

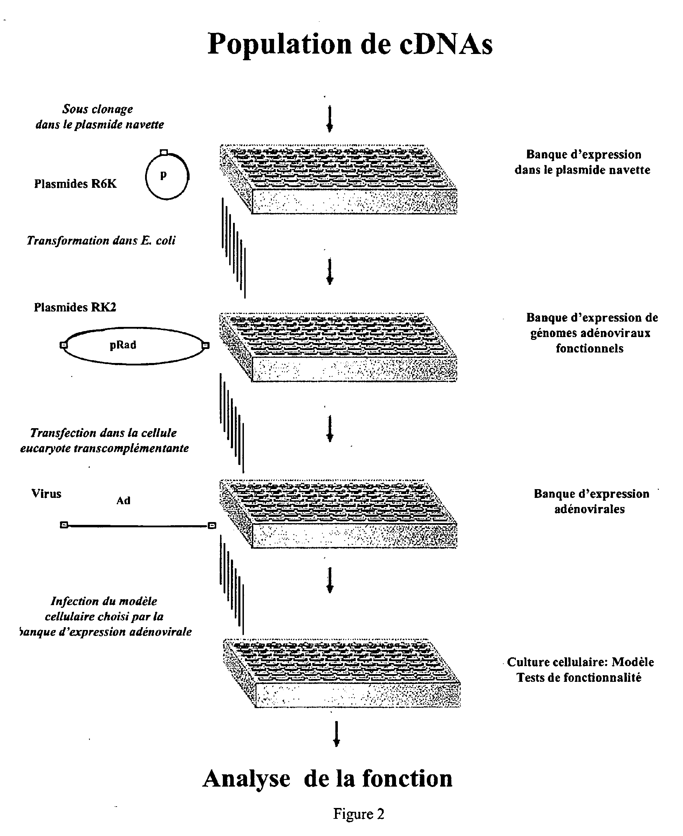

Avantageusement, pour permettre des études de fonctionnalité plus robustes, les clones sont traités séparément et non en pool. Les clones de la banque primaire dans le plasmide navette sont ainsi transformés individuellement en microplaques dans la bactérie possédant déjà le plasmide adénoviral tronqué parental. Par croissance en microplaque sous pression de sélection (antibiotiques), les ADN produits sont purifiés et transfectés dans la cellule eucaryote productrice après excision des génomes par l'enzyme choisie (par exemple Pacl). Un tel procédé est schématisé figure 2.

Selon un mode de réalisation préféré du procédé de préparation de banques adénovirales d'expression de la présente invention, on peut utiliser la technologie de clonage Gateway^{®} qui a été développée par Life Technologies, Inc. (LTI, Rockville, MA, USA) et permet notamment de générer simultanément un ensemble d'adénovirus porteurs d'inserts issus par exemple d'une banque d'ADN complémentaire ou d'un groupe de séquences caractérisées et sélectionnées par différentes méthodologies bien connues de l'homme du métier.

La technologie de clonage Gateway^{®} qui est entre autre décrite dans le brevet US 5,888,732, permet de transférer des inserts entre par exemple deux vecteurs plasmidiques, lors d'une réaction in vitro, grâce à des réactions de recombinaison au niveau des sites spécifiques *att*L, *att*R, *att*B, et *att*P du bactériophage lambda (λ) d'Escherichia coli. L'insertion d'un gène ne nécessite plus la présence de sites de restriction appropriés au sein du vecteur, mais seulement la présence de séquences courtes de recombinaison appelée séquences *att.*

Le système de clonage Gateway^{®} consiste d'une part à effectuer une réaction dite LR, au cours de laquelle un clone d'Entrée, contenant un insert d'intérêt entouré des sites *att*L, recombine avec un clone Destination contenant des sites *att*R, afin de générer un clone d'Expression. L'insert d'intérêt qui est porté par le clone Entrée est alors transféré dans un vecteur d'Expression, provenant du clone Destination, contenant les sites *att*B. Le système Gateway^{®} consiste d'autre part à effectuer la réaction inverse, qui est désignée réaction BP, au cours de laquelle le clone d'Expression contenant un insert d'intérêt entouré des sites de recombinaison *att*B recombine avec un vecteur Donneur contenant les sites *att*P, pour donner le clone d'Entrée, qui contient les sites *att*L.

Plus particulièrement, on peut modifier selon une première stratégie, un plasmide navette par exemple pJJ1, tel que décrit précédemment, afin de générer un plasmide compatible avec le système Gateway^{®}, qui possède les sites *att* appropriés. Selon cette première approche, les sites *att* sont de préférence introduits entre le promoteur (CMV) et le site de polyadénylation de SV40 de la cassette d'expression du plasmide navette. Le plasmide ainsi obtenu est désigné plasmide Destination selon la terminologie de la technologie Gateway^{®}, et peut ensuite entrer en réaction avec les clones Entrée de la technologie Gateway^{®}. En conséquence, tout insert issu d'une banque d'ADN ou d'un groupe de séquences sélectionnées, qui est cloné dans un vecteur ou clone d'Entrée peut être transféré dans le plasmide navette au sens de l'invention pour donner un plasmide navette d'Expression utilisable dans l'invention pour générer le vecteur adénoviral correspondant.

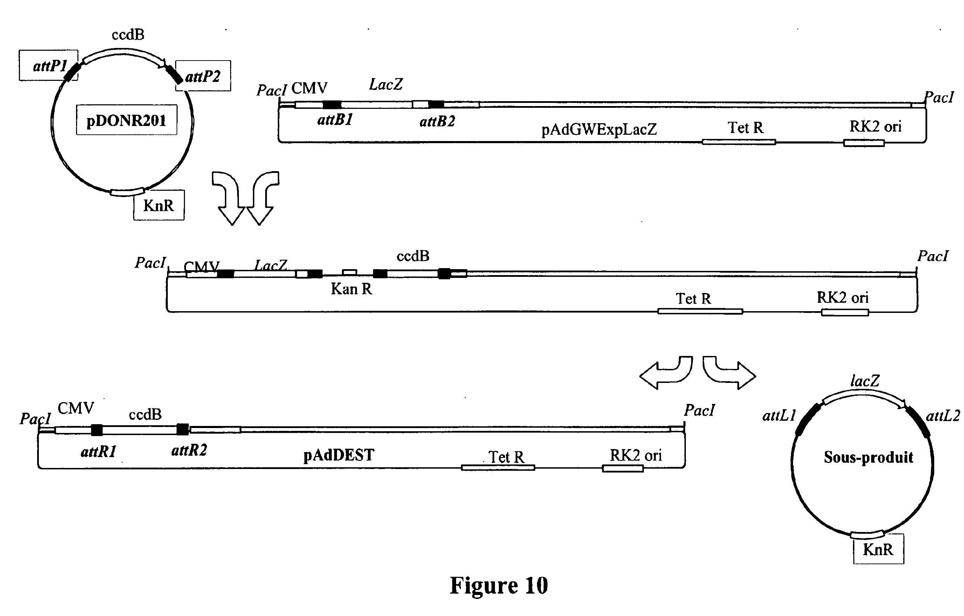

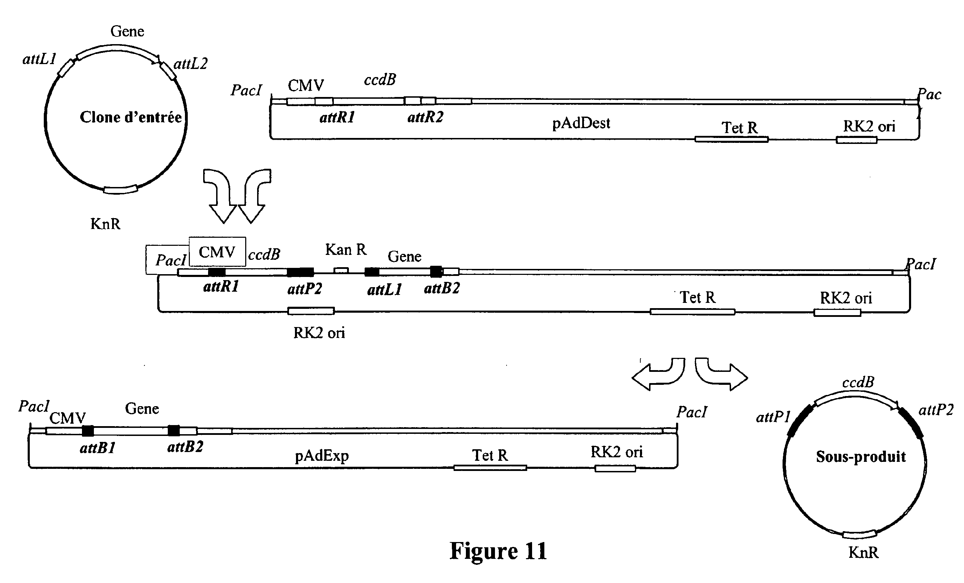

Selon une seconde stratégie, le plasmide adénoviral complet est modifié pour être compatible avec la technologie Gateway^{®}. Il s'agit donc d'introduire les sites *att* appropriés au sein du plasmide porteur d'un génome adénoviral afin de permettre la réalisation des réactions décrites dans le système de clonage Gateway^{®}. De préférence, les sites *att* sont introduits en lieu et place de la région E1 du génome adénoviral entre un promoteur et une séquence de polyadénylation. Cette stratégie permet ainsi de générer des plasmides porteurs de génomes adénoviraux comprenant des inserts variés originellement présents dans des clones d'Entrée. Il peut s'agir d'inserts issus de la fabrication de banques d'ADN complémentaires, d'inserts représentants les gènes d'une même famille ou de toutes autres origines. On génère un plasmide porteur d'un génome adénoviral, modifié et possédant les séquences *att,* qui est un clone Destination au sens de la technologie Gateway^{®}, et est désigné pAdDEST (Exemple 6, figure 10). Le clone Destination ainsi obtenu, pAdDEST, peut réagir avec tout clone d'Entrée pour générer un plasmide d'Expression porteur du génome adénoviral : pAdExp comme décrit ci-après dans l'Exemple 6 et à la figure 11.

L'invention représente ainsi une nouvelle méthode pour valider ou identifier une séquence associée à un effet physiologique ou génétique induisant un phénotype caractéristique. Il peut s'agir plus particulièrement d'un gène provoquant un défaut génétique, ou d'un gène qui pourrait être indicatif ou caractéristique d'une anomalie génétique.

Dans une application de validation ou de détermination de la fonction d'une ou plusieurs séquences dans le cadre d'une activité de génomique fonctionnelle, les séquences peuvent être de toutes natures, en particulier celles citées plus haut, mais également provenir de populations de toutes séquences de nature inconnues et multiples. Ces séquences peuvent êtres juxtaposées en différentes cassettes pour être ultérieurement introduites dans le génome adénoviral souhaité.

Ces séquences peuvent être issues de tout type cellulaire vivant ou produite artificiellement par différents procédés comme la synthèse de novo, la mutagenèse, la combinaison de séquences etc.

Un grand nombre de banques peuvent être utilisées comme source de séquences nucléotidiques dans le cadre de la présente invention, incluant mais non limité à des banques d'ADN génomique (en particulier de régions de chromosomes - inclus par exemple dans des YACs ou des BACs), des banques d'ADNc, des banques d'ADN synthétiques, que ces banques soient normalisées ou non et préparées à partir de différents tissus vivants pour de l'expression sens ou antisens, de séquences générées au hasard ayant le pouvoir de générer des peptides variés, de séquences dégénérées à partir d'une séquence connue, d'une séquence mosaïque de séquences connues, etc.

En particulier, les séquences primaires à étudier peuvent générer des antisens ou des éléments de suppression génétique. Il s'agit de séquences qui s'exprimant peuvent inactiver un gène particulier dans le système biologique étudié. Il peut s'agir de ribozyme.

Les séquences utilisées pour former la banque peuvent provenir de différents processus de synthèse. En particulier, la simulation d'une évolution moléculaire dirigée qui utilise la recombinaison permet de générer une diversité de gènes en utilisant des mécanismes identifiés de l'évolution (Curr. Op. in Biotech. 1997, 8: 724-733).

Les séquences utilisées pour former la banques peuvent aussi provenir de l'analyse bio-informatique des banques de données qui recensent les données du génome humains. Elles peuvent être sélectionnées en utilisant l'analyse de l'expression génique obtenues par différentes méthodes ( EST, Microarrays, DNA chips, differential display) TIBtech 1996, 14 p294-298; Nature Biotechnology 1998, 16, p27-31.

En particulier, le procédé permet de générer une banque adénovirale d'expression de séquences de peptides randomisées. Il permet alors de faciliter la sélection et l'identification de séquence de peptides aléatoires capables de se lier à une protéine d'intérêt. Une banque d'oligonucléotides de séquence aléatoire est introduite dans un site (polylinker) correspondant à une boucle flexible d'une protéine naturelle ou synthétique pour que les peptides soient présentés à la surface de la protéine. Il peut s'agir de présenter ces peptides de façon intracellulaire (Colas et al, 1996, Nature 380: 548-550) ou à la surface de la cellule (Tramonto et al, 1994, J.Mol.Recognit. 7:9-24). Enfin les peptides peuvent être présentés à l'extérieur de la cellule en insérant un signal de sécrétion dans la cassette d'expression (Rice et al, 1992, PNAS 89, 5467-5471).

Une ou plusieurs séquences d'intérêt peuvent être intégrées au vecteur.

Il peut s'agir d'évaluer au final une combinaison de séquences (cassette d'expression) ou de co-exprimer la séquence d'intérêt avec un autre gène d'intérêt, par exemple un gène marqueur. On peut alors décider soit d'utiliser des séquences polycistroniques soit de placer le ou les séquences à exprimer en une localisation différentes de la séquence d'intérêt au sein du génome adénoviral. Les séquences polycistroniques utilisables sont variées. Les éléments IRES permettent la traduction efficace de deux ou plus phases ouvertes de lectures à partir d'un seul ARNm. Plus particulièrement, une phase de lecture code pour la protéine d'intérêt (provenant d'une banque d'ADNc) l'autre phase de lecture pour un marqueur comme la GFP.

Le procédé permet d'utiliser les systèmes d'excision/intégration liées à une activité enzymatique. Il peut s'agir du système CRE-Lox (Baubonis et al, Nucl. Acids Res. 21:2025-2029) du système FLP recombinase-FRT ( Dang et al., Dev. Gent. 13; 367-375) du système Piv de Moraxella lacunata (Lenic et al. 1994, J. Bacteriol. 176 -4160) ou du système de l'intégrase lambda ( Kwon et al. 1997 Science 276, 126). Plus particulièrement, une séquence ou la cassette d'expression de l'ADNc est jointe à un vecteur épisomal replicatif est excisé par le système Cre-Iox du génome adénoviral et sera transmise aux cellules infectées qui se diviseront (WO97/47757).

Le procédé facilite la mise en évidence des interactions ADN-proteines. La banque correspond alors à une population de séquences capables de se lier avec l'ADN. Il s'agit de polypeptides issus de protéines capables naturellement de se fixer à l'ADN ou de polypeptides artificiels. On recherche la séquence la plus spécifique d'une séquence ADN donné.

Le procédé permet la mise en évidence d'interactions Protéines-Protéines. La capacité de co-infection du vecteur adénoviral est utilisé. Préférentiellement le test de complémentation LacZ est employée (PNAS 1997, 94, 8405-8410) . Le système cellulaire est co-infecté par la première construction et la banques d'ADNc

Les tests de fonctionnalités qui peuvent être mis en oeuvre pour analyser la banque d'expression adénovirale de l'invention sont nombreux et variés.

On peut utiliser par exemple des tests de complémentation qui consistent à identifier la séquence nucléotidique issu de la banque dont l'expression conduit à un phénotype cellulaire recherché. Plus particulièrement, la banque adénovirale d'expression permet d'infecter une cellule humaine déficiente pour un trait phénotypique. Analyse des cellules infectées et repérage des cellules qui arborent alors le trait phénotypique recherché. Analyse de l'ADNc porté par le virus responsable de l'apparition du trait phénotypique. On peut également mesurer la disparition d'un tait phénotypique après infection résultant d'une activité antisens ou d'un « knock-out » fonctionnel.

Le procédé facilite l'étude du mode d'action d'une molécule chimique dans un système biologique. La banque d'expression adénovirale sert à infecter les cellules du système biologique répondant à l'action de la drogue. Après infection, l'effet de la drogue peut être non modifié, augmenté ou inhibé. Dans les deux derniers cas, l'analyse des cDNA portés par les génomes viraux facilite la découverte des voies intracellulaires et/ou extracellulaire impliquées dans l'effet du composé chimique.

Le procédé de l'invention peut ainsi être mis en oeuvre pour
- la recherche d'acides nucléiques (cibles) permettant de contourner les effets de p53 dans le processus d'arrêt de croissance et d'apoptose.
- l'identification de cytokines
- l'identification de peptides synthétiques qui affectent les processus cellulaires
- la recherche de molécules capables d'effectuer une présentation extracellulaire de peptides
- l'identification de cibles pour des cellules tumorales qui ne présentent pas de suppresseurs de tumeurs spécifiques.
- L'identifications de gènes impliqués dans le processus de métastases.

L'invention est également relative à un kit comprenant :
- un plasmide navette comprenant un premier génome tronqué d'adénovirus, et
- un plasmide parent comprenant un deuxième génome tronqué d'adénovirus,
les deux plasmides étant susceptibles de produire, par recombinaison homologue entre les deux génomes tronqués d'adénovirus, un plasmide final comprenant un génome d'adénovirus recombinant linéaire complet, bordé par un ou plusieurs site de restrictions non présent dans ledit génome.

La présente invention sera plus complètement décrite à l'aide des exemples qui suivent, qui doivent être considérés comme illustratifs et non limitatifs.

### LEGENDE DES FIGURES

Figure 1 Schéma descriptif du principe de l'invention. Un plasmide navette pJJ1 et un plasmide parental pOSE1 possédant chacun un fragment complémentaire forment un co-agrégat par recombinaison homologue chez E coli générant un génome complet d'adénovirus recombinant portant une séquence exogène.
   KmR: gène de résistance à la Kanamycine. Tet R: Gène de résistance à la tétracycline. RK2 Ori: Origine de réplication du plasmide RK2. RK6 Ori: Origine de réplication du plasmide RK6
Figure 2 Génération simultanée de 96 adénovirus recombinants portant des cDNA issus d'une banque cDNA construite dans le plasmide navette. Application des propriétés de la technologie OSEDRAG.
Figure 3 Obtention du plasmide parental au terme de l'invention portant une séquence de génome adénoviral non-complète et donc non fonctionnelle par la technologie EDRAG (Crouzet et al.). Carte des plasmides pJJ301, pXL3215 et du plasmide pOSE17-00.
Figure 4 Cartes de différentes versions de plasmides parentaux utilisables par l'invention pour générer différentes versions de vecteurs adénoviraux recombinants, en particulier des vecteurs délétés pour E1 et E3 (pOSE 10-00), des vecteurs délétés pour E1 et E3 et munis de la région pIX sous sa forme syngen#2 (WO99/25861) -(pOSE17-00), des vecteurs délétés pour E1,E3 et E4 ( pOSE 30-00) et des vecteurs délétés pour E1, E3 et E4 et munis et munis de la région pIX sous sa forme syngen#2 (pOSE37-00).
Figure 5 Obtention à l'aide du procédé d'invention d'un génome adénoviral recombinant portant une cassette d'expression du gène LacZ. Carte du plasmide navette pIG5 et du plasmide parental pOSE17-00 et du plasmide final pAd17-01. Carte des plasmides pIG5, pOSE17-00 et pAd17-01.
Figure 6 Obtention à l'aide du procédé d'invention de génomes adénoviraux à l'aide de trois plasmides navette différents (plG5: porteur d'une cassette d'expression du gene LacZ; du plasmide pIG18: porteur d'une cassette d'expression pour le gène luciférase et plG7: sans cassette d'expression). Digestion enzymatique des clones résultants de la co-transformation du plasmide navette et du plasmide parental.
Figure 7 Obtention à l'aide du procédé d'invention des vecteurs adénoviraux suite à la transfection de différents clones du plasmide pAD17-01 dans la cellule productrice 293. Digestion enzymatique des ADN viraux purifiés à partir des virus amplifiés dans la cellule 293.
Figure 8 Obtention à l'aide du procédé d'invention d'une banque de génomes adénoviraux générés par une banque primaire d'ADNc dans le plasmide navette pTA00. La banque de génomes adénoviraux est obtenue grâce au plasmide parental pOSE37-10 qui est un plasmide dérivé de pOSE37-00. Carte des plasmide pTA00, pOSE37-10 et pAd37-10.
Figure 9 Obtention d'un plasmide d'expression pAdGWExpLacZ compatible avec le système Gateway® (LTI, Rockeville, MA, USA) par recombinaison homologue des plasmides pSHExpLacZ et PxI2689.
Figure 10 Obtention par une réaction dite BP d'un plasmide Destination pAdDEST qui porte un génome adénoviral complet, le gène ccdB létal pour *E. Coli,* entouré des sites de recombinaison *att*R1 et *att*R2.
Figure 11 Obtention du plasmide adénoviral porteur d'un insert d'intérêt pAdExp par réaction dite LR.

### Techniques générales de clonage, de biologie moléculaire, de biologie cellulaire.

Les méthodes classiques de biologie moléculaires telles que la centrifugation d'ADN plasmidique en gradient de chlorure de césium-bromure d'éthidium, les digestions par les enzymes de restriction, l'électrophorèse sur gel, la transformation dans E.coli, la précipitation des acides nucléiques etc, sont décrites dans la littérature (Maniatis et al., 1989).

Les enzymes ont été fournies par New-England Biolabs (Beverly, MA).
Pour les ligatures les fragments d'ADN sont séparés selon leur taille sur des gels d'agarose de 0,8 à 1,5 %, purifiés par GeneClean (BIO101, LaJolla CA) et incubés de nuit à 14°C dans un tampon Tris-HCl pH 7.4 50 mM, MgCl₂ 10 mM, DTT 10 mM, ATP 2 mM, en présence d'ADN ligase du phage T4.

Les ADN ligaturés sont utilisés pour transformer la souche rendue compétente E.coli TG1 [Δ(lac proA,B), supE, thi,hsdD5/ F' traD36, proA⁺, B⁺, lacl^{q}, IacZΔM15] (Maniatis et al., 1982), la souche E.coli Top10 cells (TOP10 One Shot kit, Invitrogen, Netherlands) ou bien la souche E.coli polA SF800 (Heffron et al., 1977).
L'amplification par PCR, (Polymerase Chain Reaction), a également été réalisée selon Maniatis et al., 1989, avec les spécifications suivantes :
- Température de dénaturation 95°C, température d'hybridation 55°C, température d'allongement 72°C. Ce cycle a été répété 25 fois dans un PTC-200 Peltier Thermal Cycler (MJ Research, Massachusetts, US).

Les oligonucléotides ont été synthétisés par la société GENSET ( Evry, France). Le séquençage a été effectué par la société ESGS (Evry, France).

### EXEMPLES

### Exemple 1 : Construction de plasmides parentaux

Cet exemple décrit l'obtention d'un génome adénoviral tronqué dans sa partie 5' et délété pour les régions E1 et E3, adénovirus humain du type 5, et bordé d'un site restriction unique en sa partie 3' dans un plasmide du groupe d'incompatibilité P se répliquant chez E coli.

Les plasmides selon la présente invention peuvent être construits de manières différentes. Selon une méthode préférée, on construit le plasmide pOSE17-00 selon la méthodologie EDRAG (FR2,730,504) à l'aide des plasmides pXL3215 et pJJ301. Le plasmide pXL3215 contient la totalité du génome de l'AD (moins deux délétions dans les régions E1 et E3), et possède une cassette d'expression du gène LacZ dirigé par le promoteur RSV dans la région E1.Le plasmide pXL 3215 est un dérivé du plasmide pXL2689 et contient l'origine de réplication du plasmide RK2, le gène de résistance à la tétracycline ( J.Crouzet PNAS,1997).

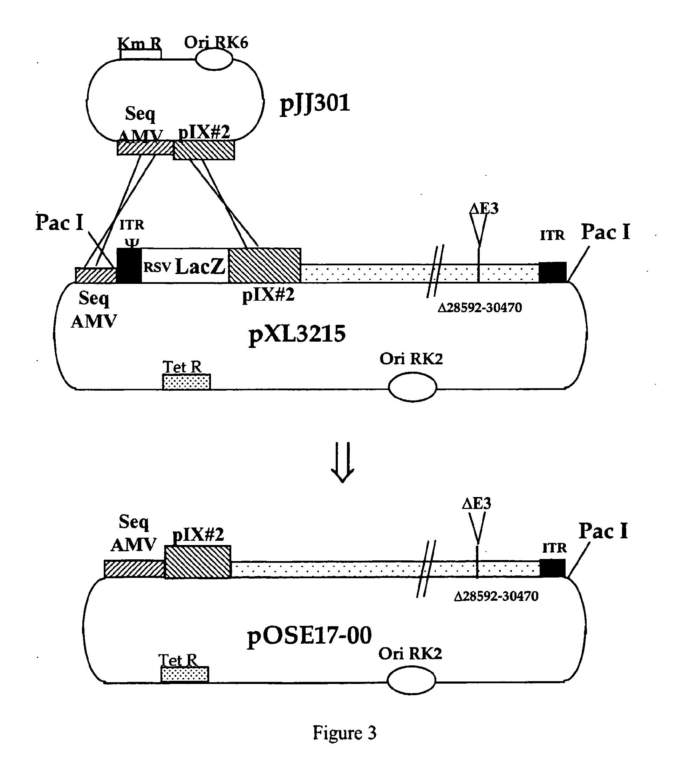

Le plasmide pJJ301 possède une origine de réplication RK6 et ne peut se répliquer dans des souches E. coli pir-. Le plasmide pJJ301 possède le gène de résistance à la Kanamycine. Le plasmide pJJ301 possède une région homologue au début du génome adénoviral porté par pOSE17-00. Il s'agit d'une version particulière de la région pIX appelée syngen#2 qui est décrite dans (WO99/25861). En amont de cette séquence est insérée une séquence homologue à la région amont du génome adénoviral complet dans le plasmide pXL3215. La double recombinaison des plasmides pXL3215 et pJJ301 donne naissance à pOSE17-00. Ce plasmide pOSE17-00 correspond à un plasmide parental au sens du procédé d'invention et est schématisé dans la Figure 3.

D'autres plasmides parentaux (pOSE 10-00, pOSE30-00, pOSE37-00) ont été construits sur le même schéma permettant de générer des génomes adénoviraux délétés dans la région E1 et E3 (en version région pIX sauvage ou version pIX dégénérée (WO99/25861) ou des génomes adénoviraux délétés dans la région E1 E3 et E4 (en version région pIX sauvage ou version pIX dégénérée (Figure 4).

### Exemple 2. Construction de plasmides navettes

Cet exemple décrit l'obtention d'un plasmide navette portant la région 5' du génome adénoviral (région ITR et d'encapsidation) ,adénovirus humain du type 5, précédée d'un site de restriction Pac I.

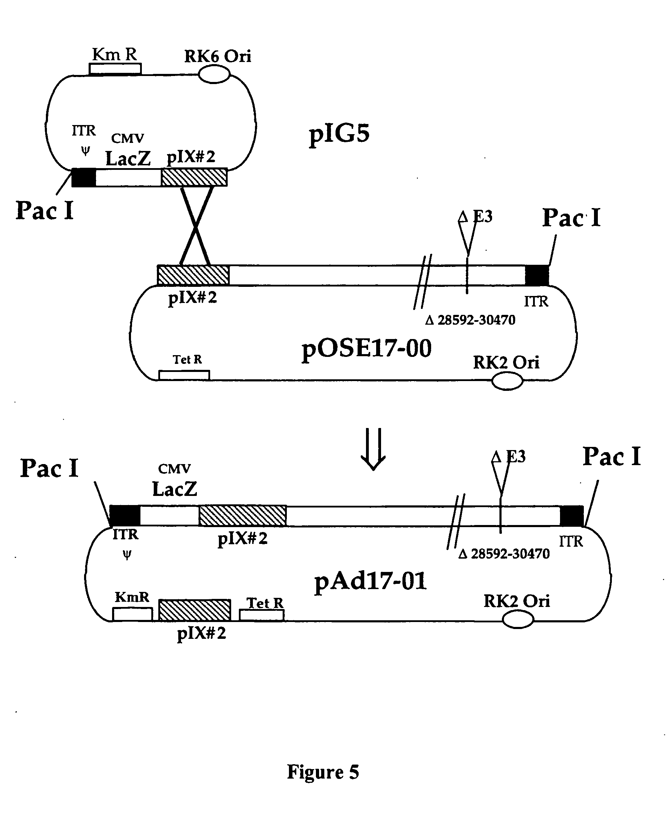

Ledit plasmide possède une origine de réplication RK6 et est incapable de se répliquer chez E coli qui ne peut transcomplémenter pour la protéine pir. Ledit plasmide pIG5 contient une cassette d'expression LacZ précédant une séquence homologue à une séquence présente dans pOSE17-00 (séquence correspondant à une partie de la région pIX Iva2).Ce plasmide est appelé un plasmide navette au sens de l'invention, il possède un gène de résistance pour la kanamycine (Figure 5).

### Exemple 3 Production d'un génome adénoviral recombinant fonctionnel

Cet exemple décrit l'obtention d'un génome adénoviral fonctionnel, délété pour les régions E1 et E3, adénovirus humain du type 5, et bordé à ses extrémités par un site restriction PacI dans un plasmide du groupe d'incompatibilité P se répliquant chez E coli et portant une cassette d'expression du gène LacZ.

La stratégie d'intégration retenue est la recombinaison homologue dans la souche E. coli entre pOSE17-00 et PIG5. Le plasmide pOSE17-00 a été introduit dans des cellules de la souche E. coli JM83 Rec⁺ IacZ⁻. A leur tour, les cellules issues d'un clone tétracycline résistant ont été rendues compétentes, transformées par le plasmide pIG5, puis étalées sur milieu LB en présence de tétracycline et de kanamycine. Etant donné que le plasmide pIG5 ne se réplique pas dans la souche JM83, l'acquisition des résistances à la tétracycline et à la kanamycine ne peut se produire que par un évènement de recombinaison homologue entre les deux plasmides. En effet ceux ci ont en commun la région pIX du génome de l'Ad5. Le plasmide résultant possède le génome complet du vecteur adénoviral recombinant bordé par un site Pacl. Des digestions par les enzymes Pacl et Hindlll, EcoRI ou encore PacI et DraI, permettent de contrôler l'obtention de la structure plasmidique attendue. Ce plasmide final portant un génome adénoviral fonctionnel a été nommé pAd17-01 (Figure 5).

De la même manière, le plasmide plG5 et le plasmide pOSE37-00 donnent naissance à un plasmide portant un génome adénoviral complet délété pour E1,E3, E4 et munis d'une région pIX sous sa forme syngen#2 (WO99/25861).

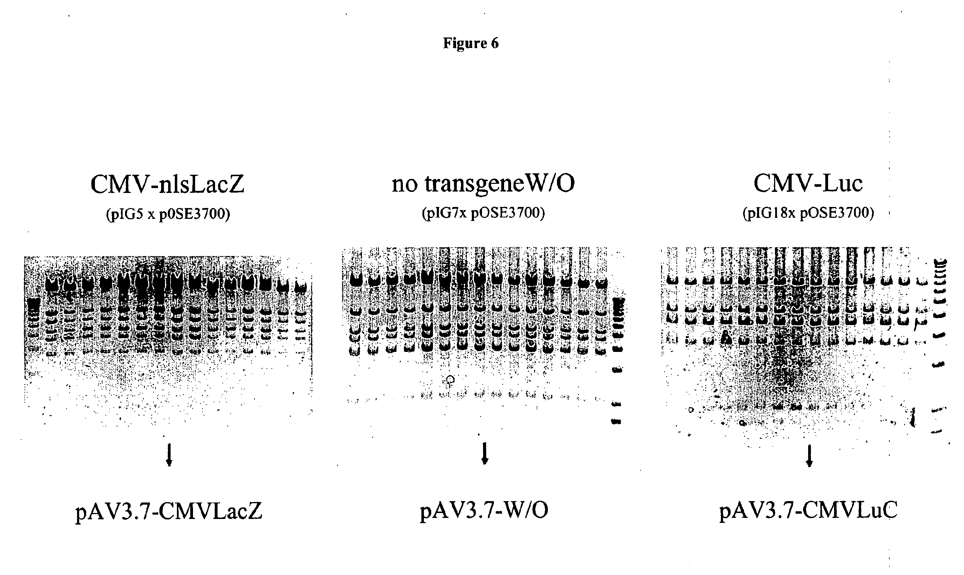

Par ailleurs, selon la même stratégie, il est possible de construire d'autres génomes recombinants de la même famille portant ou non une cassette d'expression. pIG7 et pIG18 sont utilisés et dérivent de pIG5 : PIG7 n'a pas de cassette d'expression, pIG18 possède une cassette d'expression du gène Luciférase. Dans la mesure ou la cassette d'expression ne présente pas d'homologie de séquence avec la séquence génomique du chromosome bactérien 100% des clones sont attendus être positifs. Comme illustré dans la figure 6, 100% des clones analysés à l'issu de ces expérience se sont en effet avérés présenter les structures nucléotidiques attendues (Figure 6).

pAd17-01 a été digéré par PacI libérant le génome de l'adénovirus recombinant. Le produit de cette digestion peut être utilisé tel quel pour la transfection de cellules de mammifères transcomplémentantes (cellules 293) pour les fonctions E1 de l'adénovirus.

### Exemple 4 : Production d'adénovirus recombinants

Des clones d'adénovirus recombinants peuvent être construits chez Escherichia coli, par (i) insertions de fragments contenant un ou plusieurs gènes avec des signaux de régulation appropriés pour exprimer ces gènes dans les cellules de mammifères étudiées, ou (ii) par délétion de certains fragments du génome de l'adénovirus, ou encore par la combinaison de ces deux événements, puis ensuite, après transfection de cellules productrices, un stock d'un tel virus recombinant peut être obtenu.

Le plasmide pAd17-01 est purifié à partir d'une culture de cellules E. Coli DH5 compétentes transformées. Le génome adénoviral est libéré par digestion en présence de l'enzyme Pacl. Le produit de digestion est utilisé directement pour produire les adénovirus recombinants. Pour cela, les cellules de la lignée 293 sont transfectées par le produit de digestion de pAd17-01 en présence d'Effectene (Qiagen, Allemagne). L'adénovirus recombinant est amplifié dans la lignée cellulaire 293, ce qui conduit à un surnageant de culture contenant l'adénovirus recombinant non purifié ayant un titre d'environ 10¹⁰ pfu/ml.

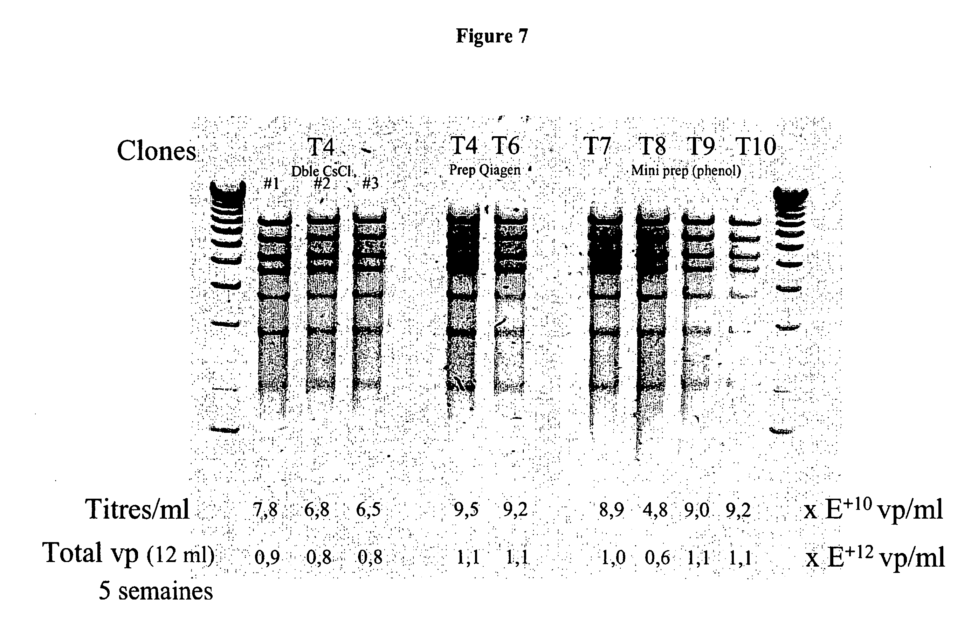

La conformité de l'ADN viral est vérifié par digestion enzymatique après purification de l'ADN issu des virus amplifiés (Figure 7).

Les particules virales sont ensuite purifiées par centrifugation sur gradient de chlorure de césium selon les techniques connues (voir notamment Graham et al., Virology 52 (1973) 456), ou par chromatographie). L'adénovirus peut être conservé à - 80°C dans 20 % de glycérol.

### Exemple 5. Construction de banques adénovirales d'expression.

On utilise le plasmide navette muni de l'origine de réplication RK6 pour obtenir une banque primaire de plasmides navettes qui servira, via la recombinaison homologue, à obtenir une banque de génomes d'adénovirus recombinants dans le plasmide possédant l'origine de réplication RK2. Cette banque de plasmides de génomes d'adénovirus recombinants sera transfectée dans la cellule productrice transcomplémentante pour obtenir une banque d'adénovirus recombinants.

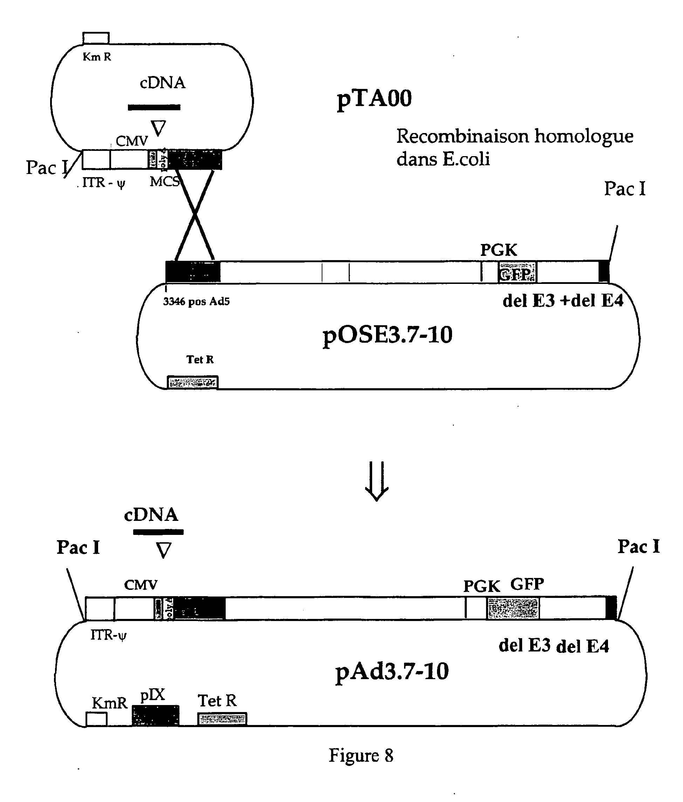

L'ensemble des étapes est schématisé en Figure 2 et les structures plasmidiques permettant l'obtention de la banque adénovirale d'expression sont présentées Figure 8 .

Exemple 5.1 Préparation de la banque d'ADNc dans le vecteur navette.

Pour la préparation de la banque dans le vecteur navette ( un dérivé de pIG5 où le gène LacZ a été excisé et remplacé par des sites multiples de clonage, en particulier les sites Xhol et EcoRI), 10-20 µg de vecteur purifié par gradient de Césium est digéré par 5U/µg des enzymes Xhol et EcoRI pendant 90 minutes à 37°C. La digestion est déposée sur un gel d'agarose (1 % Seakem GTG), et le vecteur linéarisé est séparé par électrophorèse dans du tampon TAE. La bande correspondant au vecteur est découpée après visualisation sur table UV. Le vecteur est élué de l'agarose (Qiaquick DNA Cleanup System, Quiagen, Allemagne) et purifié par une extraction phénol/chloroforme suivie d'une précipitation à l'éthanol. Ce vecteur permet d'obtenir, après ligation d'un insert test digéré par EcoR1 et Xho1, environ 5 millions de clones /µg avec un bruit de fond inférieur à 10%.

Préparation des ADNc : La synthèse d'ADNc est obtenue à partir d'une population d'ARN enrichie en mRNA.La synthèse du premier brin se fait suivant les protocoles classiques en utilisant la transcriptase Superscript Il (Stratagène) à l'aide d'un primer oligo dT possédant la séquence d'un site Xhol en5'. La synthèse du deuxième brin est effectuée par l'enzyme ADN polymérasel de E. coli. en présence de RNAse H et de ligase ADN d' E.coli. Les extrémités générées par le second brin sont remplies par l'action de la polymérase ADN T4.

Les ADNc sont fractionnés selon la taille par chromatographie par filtration sur gel à l'aide de Biogel A50M comme précédemment décrit ( Soares et al. PNAS, 91:9228-9232). Les ADNc fractionnés par la taille sont ligaturés à des adaptateurs commerciaux EcoRI (Stratagène), puis digérés par EcoRI pour créer des fragments d'ADNc munis d'extrémités EcoRI (5') et Xhol (3'). Les adaptateurs non ligaturés sont éliminés par chromatographie sur colonne de Sépharose CL4B (Pharmacia). Les ADNc portant les adaptateurs sont phosphorylés en utilisant la kinase de polynucléotides T4 et sont ligaturés en utilisant la ligase ADN T4 dans les sites EcoRI, Xhol du vecteur navette préparé comme décrit plus haut, à 16°C sur une période allant jusqu'à 48h (600ng vecteur +300 ng insert dans un volume de 10-20 µl). La banque est amplifiée par électroporation dans la souche pir.116+ permettant la réplication de plasmides munis d'une origine de réplication RK6, puis étalée sur 100 boites de 150 mm de diamètre en milieu Agar-LB, plus de la kanamycine. Une banque de 5 millions de clones est obtenue. La banque primaire d'ADNc dans le plasmide navette est ensuite normalisée, selon le protocole décrit par Soaes et al. 1994. Une étape de sélection d'un sous-ensemble de clones peut être réalisée, en s'appuyant sur les technologies permettant de déterminer le profil d'expression des clones, comme par exemple au moyen de puces à AND (Amersham, Molecular Dynamics, Affymetrix).

Les clones sélectionnés issus de la banque d'ADNc normalisée dans le plasmide navette sont ordonnés dans des microplaques de 96 puits profonds à raison d'un clone par puit. Ces clones sont amplifiés en milieu liquide (LB) en présence de l'antibiotique adapté (Kanamycine). La préparation de l'ADN de chaque clone est effectuée simultanément pour 96 échantillon de culture à l'aide du robot Quiagen (Quiagen Biorobot 9600) et du kit Quiaprep 96 Turbo Biorobot (Quiagen, Allemagne). Les ADN sont repris dans 50 µl de TEx1. 96 échantillons d'ADN plasmidiques sont ordonnés par plaque dite primaire.

### Exemple 5.2 Préparation de la banque plasmidique de génomes adénoviraux fonctionnels portant les ADNc.

Le génome adénoviral parental porté par un plasmide RK2 ( pOSE37-00) est introduit par transformation classique dans la souche JM83. Une culture de cette souche munie du plasmide (JM83xpOSE37-00) est rendue compétente par les techniques standards par lavages successifs en CaCl2 100mM.

A raison de 40 µl par puit, les cellules compétentes (JM83xpOSE37-00) sont distribuées dans une microplaque 96 puits profonds. L'ADN de chaque plasmide navette est transformé dans cette souche pour obtenir l'événement de recombinaison homologue générant le génome adénoviral fonctionnel. 5 µl d'ADN issu de la plaque dite primaire sont alors ajoutés au 40 µl de cellules compétentes en respectant l'ordonnancement. La plaque est portée à 42°C pendant 1 min puis remise sur glace pendant 2 min. 1 ml de milieu (LB) contenant les deux antibiotiques adaptés ( Kanamycine, Tétracycline) est ajouté par puits. Après une première pousse de 6h, 50 µl de cette pousse servent à ensemencer une nouvelle plaque 96 puits profonds munis d'1 ml de milieu (LB) contenant les deux antibiotiques adaptés (Kanamycine, Tétracycline). La culture est maintenue 14h.

L'ADN plasmidique résultant de cette pousse est purifié à l'aide du robot Quiagen (Quiagen Biorobot 9600) et du kit R.E.A.L Prep 96 Biorobot, auquel est ajoutée une étape de séparation Quiawell issu du kit Qiawell 96 Ultra Biorobot Kit avant la précipitation en isopropanol (Quiagen, Allemagne). L'ADN est repris dans 50 µl de TEx1.

20 µl de chaque ADN (environ 0,4 µg) sont alors transportés dans un puits d'une nouvelle micro plaque 96 puits en respectant l'ordonnancement. Cet ADN est digéré par l'enzyme PacI pour exciser le génome viral. Par puits, 10 µl de mélange réactionnel (3 µl de tampon Ne1, 6 µl H20, 1 µl (2.5 U) de Pacl) sont ajoutés au 20µl d'ADN. La réaction s'effectue à 37°C pendant 1 h30. La plaque est centrifugée et congelée à -20°C jusqu'à l'étape suivante.

L'ADN viral doit être digéré par Pacl pour exciser le génome adénoviral, puis transfecté dans les cellules eucaryotes transcomplémentantes pour générer la banque virale.

### Exemple 5.3. Obtention de la banque adénovirale d'expression.

La plaque 96 puits portant les ADN digérés par Pacl est amenée à température ambiante. Les 30 µl d'ADN digéré par PacI de chaque puits reçoivent 2µl d'Enhancer et 15 µl d'effectene (Effectene Transfection Reagent, Quiagen, Allemagne). Le mélange transfectant est alors distribué dans des puits d'une plaque 96 ou 48 puits ou ont été ensemencées des cellules IGRP2 (WO96/22378) à raison de 10⁵ cellules par cm².

14 Jour après la transfection, 50-75 µl / puits de surnageant de la culture est prélevé et sert d'inoculum pour infecter une nouvelle plaque de cellules transcomplémentantes fraîchement ensemencées. Cette étape d'amplification est renouvelée 3-5 fois pour assurer une homogénéité des titres viraux obtenus dans chacun des puits. Le titre obtenu dans chacun des puits est compris entre 5x10⁸ et 5x10⁹ particules virales par ml. La plaque est centrifugée et congelée à -20°C.

Ces virus peuvent être utilisés directement sur le système biologique approprié. Après infection du modèle cellulaire par la banque, une analyse fonctionnelle est déterminée et mise en oeuvre. On recherche plus préférentiellement une activité pro-apoptotique, anti-angiogénique ou anti-apoptotique selon le modèle cellulaire utilisé. L'ordonnancement des clones puis des virus lors de la construction de la banque permet de remonter facilement à la séquence nucléotidique reliée à une activité fonctionnelle et ainsi de définir une nouvelle cible.

### Exemple 6. Construction d'un plasmide porteur d'un génome adénoviral compatible avec le système de clonage Gateway® commercialisé par Life Technologies (LTI, Rockeville, MA, USA).

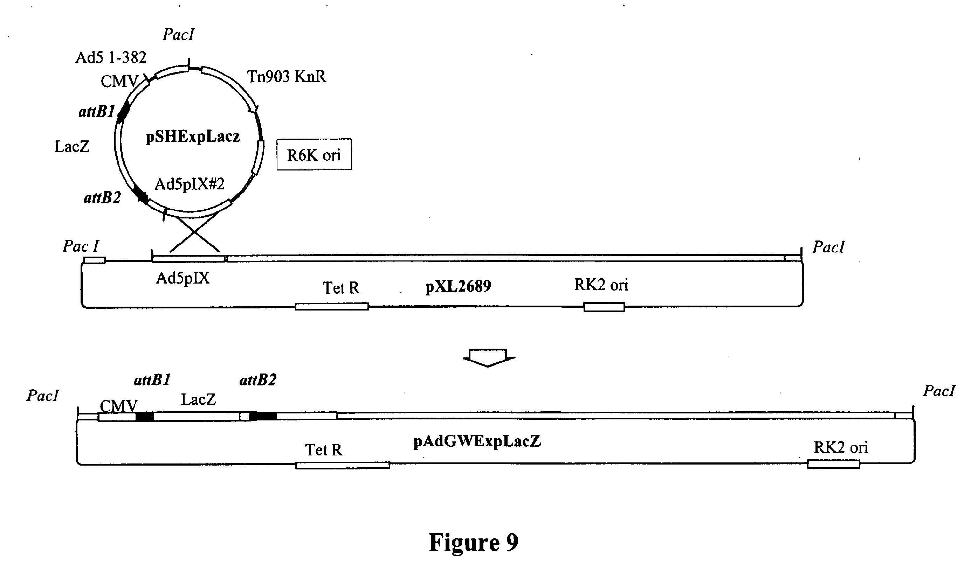

Le plasmide pSHExplacZ est généré, il possède le promoteur CMV, le site *att*B1, le gène LacZ, le site *att*B2, et le site de polyadénylation de SV40 dans un vecteur navette de type pIG5 (Figure 2). Pour obtenir ce plasmide, le cDNA du gène LacZ provenant du plasmide pSV40-lacZ (Promega) est digéré par Hindlll et DraI et inséré dans les sites Xmnl et EcoRV de pENTR-1A décrit par Life Technologies dans (US 5,888,732) donnant le plasmide pENTR-LacZ. Une réaction de type LR (Gateway^{®} cloning technology) entre pENTR-LacZ et pDest12.2 donne pExpLacZ (dénomination de la technologie Gateway^{®}). Deux étapes de sous-clonage ont facilité l'obtention finale de pSHExpLacZ : le fragment NcoI/ Asel de pExpLacZ est inséré en Asel et Ncol de pCA350 (dérivé de pIG5) générant le plasmide pSExpLacZ. Enfin, le fragment Pacl/Sall de pSExpLacZ est inséré en Pacl/Sall du plasmide pIG7 (dérivé de pIG5). Au final les séquences : promoteur CMV, site *att*B1, gène LacZ, site *att*B2, et site de polyadénylation de SV40, se retrouvent en lieu et place de la cassette CMV-LacZ du plasmide navette plG5 (Exemple 2). Le plasmide ainsi obtenu est désigné pSHExpLacZ et est représenté à la Figure 9.

Un plasmide désigné pAdGWExpLacZ compatible avec le système Gateway^{®}, est ensuite construit selon la méthodologie EDRAG (FR 2,730,504) à l'aide des plasmides pSHExpLacZ et pXL2689.

Le plasmide pXL2689 qui est décrit par Crouzet et al. (*PNAS* 1997), possède l'origine du plasmide RK2, le gène de résistance à la tétracycline, et un génome adénoviral infectieux présentant des délétions dans E1 et E3. La recombinaison des plasmides pXL2689 et pSHExpLacZ donne naissance au plasmide pAdGWExpLacZ porteur du génome adénoviral et de l'insert comprenant le promoteur CMV, le site *att*B1, le gène LacZ, le site *att*B2, et le site de polyadénylation de SV40 (figure 9).

Le plasmide pAdGWExpLacZ ainsi obtenu comprend un gène d'intérêt représenté par LacZ entre les deux sites de recombinaison *att*B du phage lambda de *E. Coli,* et réagit avec un vecteur dit donneur du système Gateway^{®} (pDONR201) portant les sites *att*P, un gène de résistance à la kanamycine (Kn^{R}), et le gène ccdB qui est létal pour *E*. *Coli.* Le plasmide résultant de cette réaction dite BP, selon la dénomination de la technologie de clonage Gateway^{®}, est désigné pAdDEST (plasmide Destination) et est représenté à la figure 10.

Enfin, le plasmide pAdDEST réagit grâce aux sites *att*R1 et *att*R2 avec un clone dit d'entrée du système Gateway^{®} portant un insert d'intérêt entouré des sites de recombinaison *att*L1 et *att*L2, par une réaction dite LR. Le plasmide ainsi obtenu est désigné pAdExp, et porte un génome adénoviral complet ainsi que l'insert d'intérêt (figure 11).

### Références bibliographiques :

**Ausubel et al., 1987.** Current protocols in molecular biology 1987-1988. John Willey and Sons, New York.
**Bolivar et al., 1977.** Gene 2:95.
**Crouzet et al., 1997,** PNAS 94, 1414-1419
**Dagert et al., 1979..** Gene, 6, 23-28.
**Ditta et al., 1980.** Plasmid, 13, 149-154.
**Ghosh-Choudhurry et al. 1986.** Gene,50 ,161-171.
**Hamilton et al., 1989.** J. Bacteriol. 171:4617-4622.
**Hanahan, D. 1983.** J. Mol. Biol. 166:557.
**Heffron et al., 1977.** Proc.Natl.Acad.Sci. USA, 74, 702-706.
**Maniatis T., et al. 1982.** Molecular Cloning: A Laboratory Manual, Cold Spring Harbor laboratory, New York.
**Miller, et al., 1988.J.** Bacteriol. 170:2575-2583.
**Simoes, et al. 1991.** New York Acad. Sci. 646:254-258.
**Sinha N.D, et al. 1984.** Nucl.Acids Res., 12, 4539-4557.
**Slater, et al. 1993..** J. Bacteriol. 175:4260-4262.
**Viera, et al., 1982.** Gene, 19, 259-268.
**Wirth, et al. 1989.** Mol. Gen. Genet., 216, 175-177.
**Zeef, et al. 1994.** EMBO J. 13:5113-5120.

## Revendications

1. Procédé de production d'adénovirus, comprenant :
- a) la construction d'un premier plasmide (appelé « navette »), de préférence procaryote, comprenant un premier génome adénoviral recombinant tronqué comprenant au moins un acide nucléique hétérologue,
- b) la mise en contact de ce premier plasmide avec un second plasmide (appelé « parent »), comprenant un deuxième génome adénoviral recombinant tronqué, complémentaire du premier, les deux plasmides étant sous forme circulaire et permettant par recombinaison homologue, la production d'un plasmide final comprenant un génome adénoviral recombinant complet, et
- c) l'excision du génome adénoviral recombinant complet linéaire à partir du plasmide final et son introduction dans une lignée d'encapsidation, pour produire les adénovirus recombinants incorporant le génome adénoviral recombinant complet.
ledit plamide « navette » et ledit plasmide « parent » comprenant l'un et l'autre une région d'homologie adénovirale identique, comprenant l'un et l'autre une origine de réplication et un marqueur de sélection différent pour permettre la sélection de chaque élément et comprenant l'un et l'autre une séquence ITR bordée d'un site de restriction non présent dans le génome adenoviral et comprenant l'un ou l'autre une séquence d'encapsidation

2. Procédé selon la revendication 1, comprenant en outre :
-- l'infection, au moyen des adénovirus recombinants produits :
- d'un matériel biologique comprenant des cellules, dans le but d'analyser in vitro les propriétés de l'acide nucléique :
- d'une culture cellulaire in vitro ou ex vivo, dans le but de produire une protéine, polypeptide ou peptide codé par l'acide nucléique hétérologue,

3. Procédé selon la revendication 1 ou 2 pour la production de banques d'expression adénovirales.

4. Kit comprenant :
- un plasmide navette comprenant un premier génome tronqué d'adénovirus, comprenant au moins un acide nucléique hétérologue, et
- un plasmide parent comprenant un deuxième génome tronqué d'adénovirus, et comprenant au moins un acide nucléique hétérologue
les deux plasmides étant susceptibles de produire, par recombinaison homologue entre les deux génomes tronqués d'adénovirus, un plasmide final comprenant un génome d'adénovirus recombinant linéaire complet, bordé par un ou plusieurs site de restrictions non présent dans ledit génome,
ledit plamide « navette » et ledit plasmide « parent » comprenant l'un et l'autre une région d'homologie adénovirale identique, comprenant l'un et l'autre une origine de réplication et un marqueur de sélection différent pour permettre la sélection de chaque élément et comprenant l'un et l'autre une séquence ITR bordée d'un site de restriction non présent dans le génome adenoviral et comprenant l'un ou l'autre une séquence d'encapsidation

## Claims

1. Process for producing adenoviruses, which comprises:
- a) constructing a first plasmid (termed "shuttle" plasmid), preferably a prokaryotic plasmid, which comprises a first truncated recombinant adenoviral genome which contains at least one heterologous nucleic acid,
- b) bringing this first plasmid into contact with a second plasmid (termed "parent" plasmid) which comprises a second truncated recombinant adenoviral genome which is complementary to the first genome, both plasmids being in circular form and making it possible, by means of homologous recombination, to produce a final plasmid which comprises a complete recombinant adenoviral genome, and
- c) excising the complete linear recombinant adenoviral genome from the final plasmid and introducing it into an encapsidating cell line in order to produce the recombinant adenoviruses which incorporate the complete recombinant adenoviral genome;
said "shuttle" plasmid and said "parent" plasmid both comprising an identical adenoviral homology region, both comprising an origin of replication and a different selection marker for selecting each element and both comprising an ITR sequence bordered by a restriction site not present in the adenoviral genome, and one or other comprising an encapsidation sequence.

2. Process according to Claim 1, which additionally comprises:
- using the recombinant adenoviruses which have been produced to infect:
- a biological material comprising cells, for the purpose of analyzing the properties of the nucleic acid;
- a cell culture in vitro or ex vivo, for the purpose of producing a protein, polypeptide or peptide which is encoded by the heterologous nucleic acid.

3. Process according to Claim 1 or 2 for producing adenoviral expression libraries.

4. Kit which comprises:
- a shuttle plasmid comprising a first truncated adenovirus genome, comprising at least one heterologous nucleic acid, and
- a parent plasmid comprising a second truncated adenovirus genome, and comprising at least one heterologous nucleic acid,
with the two plasmids being able to produce, by means of homologous recombination between the two truncated adenovirus genomes, a final plasmid which comprises a complete linear recombinant adenovirus genome which is flanked by one or more restriction sites which is/are not present in said genome,
said "shuttle" plasmid and said "parent" plasmid both comprising an identical adenoviral homology region, both comprising an origin of replication and a different selection marker for selecting each element and both comprising an ITR sequence bordered by a restriction site not present in the adenoviral genome, and one or other comprising an encapsidation sequence.

## Patentansprüche

1. Verfahren zur Herstellung von Adenovirus, umfassend:
- a) die Konstruktion eines ersten, vorzugsweise prokaryotischen Plasmids (genannt "Fähre"), das ein erstes trunkiertes rekombinantes adenovirales Genom umfasst, das mindestens eine heterologe Nukleinsäure umfasst,
- b) das Kontaktieren dieses ersten Plasmids mit einem zweiten Plasmid (genannt "Eltern"), das ein zweites trunkiertes rekombinantes adenovirales Genom umfasst, das komplementär zum ersten ist, wobei die beiden Plasmide eine zirkuläre Form aufweisen und durch homologe Rekombination die Herstellung eines fertigen Plasmids ermöglichen, das ein vollständiges rekombinantes adenovirales Genom umfasst, und
- c) die Exzision des linearen vollständigen rekombinanten adenoviralen Genoms aus dem fertigen Plasmid und dessen Einführung in eine Enkapsidierungslinie, um die rekombinanten Adenoviren herzustellen, die das vollständige rekombinante adenovirale Genom inkorporieren,
wobei beide, das "Fähren"-Plasmid und das "Eltern"-Plasmid, eine identische adenovirale Homologieregion umfassen, beide einen Replikationsstartpunkt und einen unterschiedlichen Selektionsmarker umfassen, um die Selektion jedes Elements zu ermöglichen, und beide eine TIR-Sequenz umfassen, die von einer Restriktionsstelle eingefasst ist, die in dem adenoviralen Genom nicht vorhanden ist, und eine von beiden eine Enkapsidierungssequenz umfasst.

2. Verfahren nach Anspruch 1, ferner umfassend:
- die Infektion mittels der hergestellten rekombinanten Adenoviren:
- eines biologischen Materials, das Zellen umfasst, mit dem Ziel in vitro die Eigenschaften der Nukleinsäure zu analysieren;
- einer In-vitro- oder Ex-vivo-Zellkultur, mit dem Ziel ein Protein, Polypeptid oder Peptid herzustellen, das die heterologe Nukleinsäure kodiert.

3. Verfahren nach Anspruch 1 oder 2 zur Herstellung adenoviraler Expressionsbanken.

4. Kit, umfassend:
- ein Fährenplasmid, das ein erstes trunkiertes Adenovirusgenom umfasst, das mindestens eine heterologe Nukleinsäure umfasst, und
- ein Elternplasmid, das ein zweites trunkiertes Adenovirusgenom umfasst, und das mindestens eine heterologe Nukleinsäure umfasst,
wobei die zwei Plasmide in der Lage sind, durch homologe Rekombination zwischen den zwei trunkierten Adenovirusgenomen ein fertiges Plasmid herzustellen, das ein vollständiges lineares rekombinantes Adenovirusgenom umfasst, das durch ein oder mehrere Restriktionsstellen eingefasst ist, die in dem Genom nicht vorhanden sind,
wobei beide, das "Fähren"-Plasmid und das "Eltern"-Plasmid, eine identische adenovirale Homologieregion umfassen, beide einen Replikationsstartpunkt und einen unterschiedlichen Selektionsmarker umfassen, um die Selektion jedes Elements zu ermöglichen, und beide eine TIR-Sequenz umfassen, die von einer Restriktionsstelle eingefasst ist, die in dem adenoviralen Genom nicht vorhanden ist, und eine von beiden eine Enkapsidierungssequenz umfasst.
